# EUROPEAN PATENT SPECIFICATION

(11) **EP 4 382 094 B1**
(45) Date of publication and mention of the grant of the patent: **04.11.2026**
(21) Application number: 22863658.5
(22) Date of filing: 05.09.2022
(51) Int. Cl.: A61K 9/127, A61K 9/1271, A61K 47/69, A61K 47/61, A61K 31/192, A61K 31/195, A61K 31/22, A61K 31/366, A61K 31/40, A61K 31/47, A61K 31/505, A61K 31/616, A61P 9/10, A61K 31/405, A61K 31/4174, A61K 31/4465

(54) **ATHEROSCLEROSIS-TARGETED LIPOSOME NANOCARRIER DELIVERY SYSTEM AND PREPARATION METHOD THEREFOR**
AUF ATHEROSKLEROSE GERICHTETES LIPOSOM-NANOTRÄGER-ABGABESYSTEM UND HERSTELLUNGSVERFAHREN DAFÜR
SYSTÈME D'ADMINISTRATION DE NANOPORTEURS DE LIPOSOMES CIBLANT L'ATHÉROSCLÉROSE ET SON PROCÉDÉ DE PRÉPARATION

(30) Priority: 06.09.2021 WO PCT/CN2021/116681
(43) Date of publication of application: 12.06.2024
(60) Divisional application: 26201811.2
(73) Proprietor: Beijing Inno Medicine Co., Ltd., Beijing 100195 (CN)
(72) Inventor: MA, Qian, Beijing 100195 (CN); CHEN, Xiaoming, Beijing 100195 (CN); DENG, Tuo, Beijing 100195 (CN); WANG, Huijing, Beijing 100195 (CN)
(74) Representative: Dennemeyer & Associates S.A.
(86) International application number: PCT/CN2022/117091
(87) International publication number: WO 2023/030524

(56) References cited:
- EP-A1- 3 744 317
- WO-A1-2019/141271
- WO-A2-2014/121211
- CN-A- 105 163 720
- CN-A- 110 974 972
- CN-A- 110 974 972
- US-A1- 2007 116 753
- US-A1- 2008 026 049
- US-A1- 2008 026 049
- US-A1- 2014 356 416
- KHERADMANDI MAHSA ET AL: "Targeting Dysfunctional Vascular Endothelial Cells Using Immunoliposomes Under Flow Conditions", CELLULAR AND MOLECULAR BIOENGINEERING, SPRINGER INTERNATIONAL PUBLISHING, CHAM, vol. 13, no. 3, 4 May 2020 (2020-05-04), pages 189 - 199, XP037125222, ISSN: 1865-5025, [retrieved on 20200504], DOI: 10.1007/S12195-020-00616-1

## Description

### FIELD OF THE INVENTION

The present disclosure belongs to the technical field of pharmaceutical technology, and in particular relates to an actively encapsulating liposome nanocarrier delivery system, a method for preparing the same and uses thereof.

### BACKGROUND

Cardiovascular and cerebrovascular diseases are the number one chronic diseases with a high disability rate, high mortality rate, high medical risk and high medical expenses and the leading cause of death globally. Cardiovascular and cerebrovascular diseases account for more than 40% of all deaths of residents, and atherosclerosis-related diseases are a top priority among cardiovascular and cerebrovascular diseases. According to statistics, there are 466 million patients with atherosclerosis worldwide, including 154 million coronary heart disease patients, with 27.3 million new cases each year. There are 19 million new cases of acute myocardial infarction worldwide each year, with a mortality rate of 34% to 42%. Even if the patients survive, their relative risk of all-cause death and cardiovascular events 1 to 5 years after the myocardial infarction is at least 30% higher than that of the general population.

The Report on Cardiovascular Diseases in China (2018*)* shows that according to the data from China's 6th National Census conducted in 2010, at present, in China there are up to 11 million patients with coronary heart disease, the number of new cases exceeds 1 million each year, and the number of deaths due to cardiovascular diseases each year is as high as 3.9 million, which accounts for more than 40% of deaths of residents caused by diseases. Many studies have confirmed that atherosclerosis is the main cause of myocardial infarction and ischemic stroke in patients. These patients are also most prone to serious adverse events such as sudden death or cardiac insufficiency, and sequelae of cerebral infarction, bringing huge medical burden to family and society.

In the face of such a serious disease, however, there is still no better prevention and treatment method worldwide. The current treatment for atherosclerosis is still via systemic administration. Oral administration of drugs can only delay the progression of plaques, but cannot significantly reverse them, let alone completely cure the disease. Moreover, Chinese people have poor tolerance to statins and other drugs, which can easily induce complications such as abnormal liver function, new-onset diabetes, rhabdomyolysis, and cognitive impairment. At present, the strategy for treating the patients is still lifelong drug treatment, which cannot cure the disease and poses many side effects of drugs. For example, treatment of atherosclerosis by oral administration of statins requires intensive large doses to stabilize the plaques, while systemic treatment with large doses of statins has a risk of increased incidence of serious side effects (such as abnormal liver function, rhabdomyolysis, type II diabetes, etc.).

For existing systemic administration, usually only a very small portion of active ingredients can actually act on a lesion site after a drug enters the body. This is especially true for atherosclerosis, which is located in the blood vessel wall where the blood flow is fast, making it impossible for the drug to enter the plaque, let alone accumulate in it. This is the fundamental cause that limits the drug efficacy and produces toxic side effects of the drug. A targeted drug delivery system refers to a drug delivery system that has the ability of targeted drug delivery. After administered via a certain route, the drug contained in the targeted drug delivery system is specifically enriched in a targeted site due to particle size, electrical properties or others, or is specifically enriched in a targeted site by a carrier with a targeting probe. The targeted drug delivery system is capable of making the drug targeting to a particular lesion site and releasing active ingredients at the target lesion site. Therefore, the targeted drug delivery system may result in a relatively high concentration of the drug in the target lesion site, and a reduced dose of the drug in the blood circulation, thereby improving the drug efficacy while suppressing toxic side effects, and reducing damage to normal tissues and cells.

Liposomes are common targeted drug delivery systems. Liposomes have obvious advantages such as improving the drug efficacy, reducing the toxic side effects, improving drug bioavailability, and having strong biological safety. ZL201980001843.4 discloses a CD44-targeted liposome nanodrug prepared by passive encapsulation, and the expression status of CD44 on the surface of macrophages, monocytes, endothelial cells, lymphocytes and smooth muscle cells mainly present within vulnerable plaques and their affinity for hyaluronic acid (HA) can be used for construction of a targeted drug delivery system which targets plaques or a disease associated with the vulnerable plaques to achieve a liposome drug delivery system for diagnosing or treating the vulnerable plaques while achieving sustained release; the current methods, however, are limited to passive drug loading methods. ZL201980001833.0 discloses that a series of hydrophilic or hydrophobic therapeutic drugs and contrast agents are encapsulated in a hydrophilic lumen or a hydrophobic lipid layer through a two-step method of film hydration and probe sonication, and are further coupled to targeting ligands. This strategy belongs to passive encapsulation. Although this strategy is highly universal, the encapsulation rate is often not high enough, and there is room for further improvement in the therapeutic effect. WO2014121211A2, CN110974972A, US20080026049A1, EP3744317A1, and Kheradmandi Mahsa et al. ("Targeting Dysfunctional Vascular Endothelial Cells Using Immunoliposomes Under Flow Conditions", CELLULAR AND MOLECULAR BIOENGINEERING, SPRINGER INTERNATIONAL PUBLISHING, CHAM, vol. 13, no. 3) also disclose a series of liposome delivery systems.

In addition, studies on the preparation of liposomes from highly lipid-soluble statins show that highly lipid-soluble statins can be encapsulated in liposome bilayer membranes by using a film hydration or reverse evaporation method to achieve purposes of, among others, increasing metabolic time and improving bioavailability. Nevertheless, these methods are not suitable for statins with a certain water solubility. In order to solve these problems, the inventors of the present patent application have discovered and disclosed a method of actively encapsulating statins with a certain water solubility through an active drug loading method to achieve targeted drug delivery.

In the field of targeted nano-formulations, the particle size of formulations and the coupling density of targeting ligands are closely correlated with the therapeutic effect, but the conclusions are not uniform; in other words, for different indications there is no uniform standard for the size of nano-formulations and the surface ligand coupling concentration, and there is a great difference for different indications. As for the disease of atherosclerosis, exploration of appropriate particle sizes and ligand concentrations plays a crucial role in drug efficacy. The present patent application discloses a new drug loading process and a formula for treating the disease of atherosclerosis using specific nanostructures and specific ligand density.

### SUMMARY OF THE INVENTION

In order to overcome the defects in the prior art, the present disclosure is intended to provide an actively encapsulating liposome nanocarrier delivery system, a method for preparing the same and uses thereof. Meanwhile, the present disclosure discloses the influence of specific particle sizes and surface ligand density on drug efficacy of targeted nano drugs.

Before setting forth the content of the present disclosure, the terms used in the present application are defined as follows:
The term "targeted drug delivery system" refers to a drug delivery system that has the ability of targeted drug delivery. After administration via a certain route, the drug contained in the targeted drug delivery system is specifically enriched in the targeted site by the action of a special carrier or a target head (e.g., a targeting ligand). Currently known means for achieving targeted drug delivery include utilizing the passive targeting properties of various microparticle drug delivery systems, introducing chemical modification on the surface of microparticle drug delivery systems, utilizing some special physical and chemical properties, utilizing an antibody-mediated targeted drug delivery, utilizing a ligand-mediated targeted drug delivery, utilizing a prodrug targeted drug delivery, etc.

The term "atherosclerosis" refers to a common and most important type of vascular diseases known as arteriosclerosis, and is characterized by lesions of an affected artery starting from the intima with accumulation of lipids and complex carbohydrates, hemorrhage and thrombosis, fibrous tissue hyperplasia and calcinosis, followed by gradual degeneration and calcification of the middle layer of the artery. Atherosclerosis is thus named because of the yellow porridge-like appearance of lipids that build up in the intima of the arteries.

The term "actively encapsulating liposome" refers to the loading of certain hydrophilic or amphiphilic compounds into preformed liposomes using a transmembrane pH or ion gradient. This technique is known as active or remote encapsulation.

To achieve the above purposes, the first aspect of the present disclosure provides an actively encapsulating liposome nanocarrier delivery system, characterized in that the liposome nanocarrier delivery system includes a liposome carrier and a substance used for preventing and/or treating atherosclerosis or an atherosclerosis-related disease actively encapsulated by the liposome carrier; wherein materials of the liposome carrier comprise phospholipid and cholesterol; the liposome nanocarrier delivery system is modified with a targeting ligand which is hyaluronic acid and DSPE-PEG2000-NH2 coupled through an amide bond; and in the liposome nanocarrier delivery system, the final concentration of the targeting ligand is 10-200 µg/mL.

The liposome nanocarrier delivery system according to the first aspect of the present disclosure, wherein the materials of the liposome carrier comprise phospholipid and cholesterol; alternatively, the phospholipid is one or more selected from the following: hydrogenated soybean phosphatidylcholine, distearoylphosphatidylcholine, distearoylphosphatidylethanolamine, polyethylene glycol-distearoylphosphatidylethanolamine, distearoylphosphatidylglycerol, distearoylphosphatidylserine, dioleoylphosphatidylcholine, dioleoylphosphatidylethanolamine, dioleoylphosphatidylserine, dioleoylphosphatidylethanolamine-polyethylene glycol, dicinnamoylphosphatidylcholine, dicinnamoylphosphatidylethanolamine, dicinnamoylphosphatidylserine, dicinnamoylphosphatidylethanolamine-polyethylene glycol, dipalmitoylphosphatidylcholine, dipalmitoylphosphatidylethanolamine, dipalmitoylphosphatidylserine, dipalmitoylphosphatidylethanolamine-polyethylene glycol, dierucoylphosphatidylcholine, dierucoylphosphatidylethanolamine, dierucylphosphatidylserine, dierucylphosphatidylethanolamine-polyethylene glycol, sphingomyelin, egg phosphatidylcholine, egg phosphatidylethanolamine, and egg phosphatidylglycerol.

The liposome nanocarrier delivery system according to the first aspect of the present disclosure, wherein the mass ratio of the substance used for preventing and/or treating atherosclerosis or an atherosclerosis-related disease to the liposome carrier is 1:0.1 to 50, alternatively 1:0.2 to 30, yet alternatively 1:0.5 to 20. Optionally, the preparation of the nanocarrier delivery system further comprises a step of using the liposome carrier hydrated with a salt solution to actively encapsulate the substance used for preventing and/or treating atherosclerosis or an atherosclerosis-related disease.

The liposome nanocarrier delivery system according to the first aspect of the present disclosure, wherein the substance used for preventing and/or treating atherosclerosis or an atherosclerosis-related disease is a weakly acidic substance;
alternatively, the substance used for preventing and/or treating atherosclerosis or an atherosclerosis-related disease is one or more selected from the following: statins, fibrates, antiplatelet drugs, PCSK9 inhibitors, anticoagulant drugs, angiotensin converting enzyme inhibitors, calcium ion antagonists, MMPs inhibitors, β-receptor blockers, glucocorticoid, and pharmaceutically acceptable salts thereof, and active formulations of the drugs or substances;
yet alternatively, the substance used for preventing and/or treating atherosclerosis or an atherosclerosis-related disease is one or more selected from the following: lovastatin, atorvastatin, rosuvastatin, simvastatin, pitavastatin, pravastatin, bezafibrate, ciprofibrate, gemfibrozil, aspirin, acemetacin, sodium ozagrel, tirofiban, and pharmacodynamic fragments or pharmaceutically acceptable salts thereof.

The liposome nanocarrier delivery system according to the first aspect of the present disclosure, wherein the liposome nanocarrier delivery system can be modified with a targeting ligand, such as GAG, collagen, laminin, fibronectin, selectin, osteopontin (OPN), monoclonal antibodies HI44a, HI313, A3D8, H90 and IM7, and hyaluronic acid or a derivative of hyaluronic acid;
alternatively, the targeting ligand is selected from hyaluronic acid, serglycin, collagen, fibronectin, selectin, osteopontin (OPN), and monoclonal antibodies HI44a and IM7.

The liposome nanocarrier delivery system according to the first aspect of the present disclosure, wherein the liposome carrier is selected from small unilamellar liposomes, large unilamellar liposomes and multilamellar liposomes.

The second aspect of the present disclosure provides a method for preparing the liposome nanocarrier delivery system according to the first aspect, comprising a step of hydrating the liposome carrier by adding a salt solution and performing separation to form a solution acidity gradient between inside and outside of the liposomes.

The liposome nanocarrier delivery system according to the second aspect of the present disclosure, wherein the salt is a salt of weak acid and strong alkali;
alternatively, the anionic portion of the salt is one or more selected from the following: acetate, edetate, bicarbonate, citrate, benzoate and gluconate; and/or
the cationic portion of the salt is one or more selected from the following: calcium ions, copper ions, nickel ions, barium ions, magnesium ions and zinc ions;
yet alternatively, the salt is one or more selected from the following: calcium acetate, calcium bicarbonate, magnesium citrate and copper gluconate.

The preparation method according to the second aspect of the present disclosure, wherein the preparation method may comprise the following steps:
(1) dissolving the materials of the liposome carrier in a good solvent;
(2) adding a salt solution that forms a solution acidity gradient between inside and outside of the liposomes to the product obtained in step (1) to hydrate and disperse it into a crude lipid solution;
(3) reducing the particle size of the crude lipid solution obtained in step (2) to obtain refined liposomes;
(4) purifying the refined liposome solution obtained in step (3) to remove external ions, and forming an acidity gradient between inside and outside of the liposomes; and
(5) adding a solution of the substance to be encapsulated to the liposome solution obtained in step (4), and performing incubation to allow the drug to enter the interior of the liposomes to obtain the liposome nanocarrier delivery system.

The preparation method according to the second aspect of the present disclosure, wherein the step (1) further comprises a step of removing the solvent by evaporation to make the liposomes form a lipid membrane;
alternatively, the evaporation temperature is 40-80°C, alternatively 50-70°C, yet alternatively 50-60°C;
yet alternatively, the evaporation method is rotary evaporation;
still alternatively, the rotary evaporation is performed at a speed of 50-200 r/min, alternatively 70-120 r/min, yet alternatively 80-100 r/min.

The preparation method according to the second aspect of the present disclosure, wherein in step (1), the good solvent is one or more selected from the following: chloroform, ethanol, methanol, methylene chloride, acetone, toluene, diethyl ether, ethyl acetate, methyl acetate, acetonitrile, and dichloroethane.

The preparation method according to the second aspect of the present disclosure, wherein in step (1), the materials of the liposome carrier comprise phospholipid and cholesterol;
alternatively, the phospholipid is at a concentration of 0.1-500 mg/mL, alternatively 0.1-200 mg/mL, yet alternatively 0.2-100 mg/mL; and/or
the cholesterol is at a concentration of 0.05-200 mg/mL, alternatively 0.1-100 mg/mL, yet alternatively 0.1-50 mg/mL.

The preparation method according to the second aspect of the present disclosure, wherein in step (2), the salt solution has a concentration of 100-500 mM, alternatively 200-300 mM, yet alternatively 200-250 mM; and/or
the hydration temperature is 30-90°C, alternatively 40-80°C, yet alternatively 45-65°C.

The preparation method according to the second aspect of the present disclosure, wherein in step (3), the method for reducing the particle size is extruding the crude lipid solution through a filter membrane;
alternatively, the filter membrane is a polycarbonate membrane;
yet alternatively, the filter membrane has a pore size of 30-800 nm, such as 50-400 nm, such as 100-200 nm, such as 30 nm, 50 nm, 100 nm, 200 nm, 400 nm, 600 nm or 800 nm, alternatively 100-150 nm.

The preparation method according to the second aspect of the present disclosure, wherein in step (4), the method for removing external ions is one or more selected from the following: separation through an ultrafiltration membrane pack, dialysis separation and ion exchange.

The preparation method according to the second aspect of the present disclosure, wherein in step (5), the solution of the substance to be encapsulated is at a concentration of 0.01-100 mg/mL, alternatively 0.05-50 mg/mL, yet alternatively 0.1-20 mg/mL, still alternatively 0.1-10 mg/mL.

The preparation method according to the second aspect of the present disclosure, wherein in step (5), the incubation temperature is 20-80°C, alternatively 25-70°C.

The preparation method according to the second aspect of the present disclosure, wherein when the liposome nanocarrier delivery system is modified with a targeting ligand, the method further comprises the following step:
(6) co-incubating the targeting ligand with the liposome nanocarrier delivery system obtained in step (5) to obtain a modified liposome nanocarrier delivery system.

The third aspect of the present disclosure provides a medicine, which comprises the nanocarrier delivery system according to the first aspect, or the nanocarrier delivery system prepared by the preparation method according to the second aspect, and at least one pharmaceutically acceptable carrier.

The fourth aspect of the present disclosure provides a use of the nanocarrier delivery system according to the first aspect, the nanocarrier delivery system prepared by the preparation method according to the second aspect, and the medicine according to the third aspect in the manufacture of a medicament for preventing and/or treating atherosclerosis or an atherosclerosis-related disease.

Alternatively, the atherosclerosis-related disease is one or more selected from the following: atherosclerosis, coronary atherosclerotic heart disease (including acute coronary syndrome, asymptomatic myocardial ischemia-latent coronary heart disease, angina pectoris, myocardial infarction, ischemic heart disease, sudden death, and in-stent restenosis), cerebral atherosclerosis (including cerebral ischemic stroke and hemorrhagic apoplexy), peripheral vascular atherosclerosis (including carotid atherosclerosis, vertebral atherosclerosis, subclavian atherosclerosis, occlusive peripheral atherosclerosis, retinal atherosclerosis, renal atherosclerosis, lower extremity atherosclerosis, upper extremity atherosclerosis, mesenteric atherosclerosis and atherosclerotic impotence), aortic dissection, hemangioma, thromboembolism, heart failure, and cardiogenic shock.

A further aspect of the present disclosure provides a use of the nanocarrier delivery system according to the first aspect, the nanocarrier delivery system prepared by the preparation method according to the second aspect, and the medicine according to the third aspect in the manufacture of a medicament for treating vascular plaques; alternatively, the vascular plaques are arterial plaques; yet alternatively, the medicament is used to curb the progression of arterial plaques, reverse arterial plaques and/or reduce the volume of arterial plaques.

The present disclosure belongs to the field of targeted formulations, and relates to an active targeting liposome nanocarrier delivery system, a method for preparing the same and uses thereof, especially an innovative method for preparing an atherosclerosis-targeted actively encapsulating liposome nano drug and uses thereof, for example, in the diagnosis, prevention and treatment of atherosclerosis or an atherosclerosis-related disease.

In order to further improve the therapeutic effect of the nano drug delivery systems, increase the drug loading and encapsulation rate of the nanocarriers, and enhance the clinical transformation ability of the targeted nano drugs, the present patent application discloses a process for preparing the targeted liposome nano drugs by active drug loading, and the inventors of the present application unexpectedly found that the appropriate particle size and the ligand concentration play a crucial role in the drug efficacy. The present patent application discloses drug-loading technology, specific carrier nanostructures and specific ligand density, as well as a new method for the treatment of atherosclerotic diseases, and achieves efficient enrichment of a variety of drugs including atherosclerotic drugs, anti-inflammatory drugs and antiplatelet drugs in an atherosclerotic area.

The present patent application discloses a series of new systems, methods and processes of active drug loading of targeted liposome drugs, which can achieve a drug encapsulation rate of 90% or more, reflecting their significant advantages as drug carriers. The present patent application further verifies their excellent therapeutic effects in model animals, and extends the application of the atherosclerosis-targeted liposome drug carriers.

Alternatively, the formula of the liposomes comprises phospholipid (0.2-100 mg/mL), cholesterol (0.1-50 mg/mL), and a drug (0.1-10 mg/mL), wherein the mass ratio of the drug to the lipid materials is 1:0.5 to 1:20.

According to one embodiment of the present disclosure, the present patent application provides a liposome formulation that loads drugs through active encapsulation for the treatment of atherosclerosis, with a particle size of 50-300 nm, a phospholipid concentration of 0.2-100 mg/mL, a cholesterol concentration of 0.1-50 mg/mL, and a drug concentration of 0.1-10 mg/mL, wherein the mass ratio of the drug to the lipid materials is between 1:0.5 and 1:20. Alternatively, the substance used for preventing and/or treating atherosclerosis or an atherosclerosis-related disease is one or more selected from the group consisting of statins, fibrates, antiplatelet drugs, PCSK9 inhibitors, anticoagulant drugs, angiotensin converting enzyme inhibitors, calcium ion antagonists, MMPs inhibitors, β-receptor blockers, glucocorticoid, and pharmaceutically acceptable salts thereof, including active formulations of the drugs or substances above.

Yet alternatively, the substance used for preventing and/or treating atherosclerosis or an atherosclerosis-related disease is one or more selected from the group consisting of lovastatin, atorvastatin, rosuvastatin, simvastatin, pitavastatin, pravastatin, bezafibrate, ciprofibrate, gemfibrozil, aspirin, acemetacin, sodium ozagrel, tirofiban, and pharmacodynamic fragments or pharmaceutically acceptable salts thereof, and one or more of the pharmaceutically acceptable salts thereof.

One embodiment of the present disclosure provides a method for preparing a nanocarrier delivery system, the method comprising the following steps:
(1) dissolving phospholipid membrane materials in a good solvent, and removing the solvent by evaporation or not removing the solvent;
(2) adding a salt solution that forms a solution acidity gradient between inside and outside of the liposomes to the solution or the evaporated dried matter obtained in step (1) to hydrate and preliminarily disperse it into a crude lipid solution;
(3) reducing the particle size of the crude lipid solution to obtain refined liposomes;
(4) removing external ions from the liposome solution obtained in step (3) by purification, and forming an acidity gradient between inside and outside of the liposomes;
(5) adding a drug liquid to be encapsulated to the liposome solution obtained in step (4) to allow the drug to enter the interior of the liposomes; and
(6) assembling a targeting ligand on the surface of the liposomes based on the insertion method.

The present disclosure provides a use of an actively encapsulating liposome in the manufacture of products for the diagnosis, prevention and treatment of atherosclerosis or an atherosclerosis-related disease.

The nanocarrier delivery system of the present disclosure may have, but is not limited to, the following beneficial effects.

The present disclosure provides a process for preparing an actively encapsulating liposome nanocarrier delivery system and an optimization process thereof, and meanwhile verifies the ability of the obtained targeted delivery system to reverse plaques. The system has high encapsulation rate and drug loading, provides strong protection for encapsulated drugs, allows no release of drugs during long-term storage, enables a slow release of drugs in the body, can increase the local enrichment concentration of the drug in a lesion, and has advantages of low toxicity and high efficiency. The method of the present disclosure is simple, green and controllable, leads to high drug loading, good compatibility with various drugs, good biocompatibility, has high safety, and enables easy implementation of targeting ligand modification, and the obtained targeted drugs have good effects on the treatment of atherosclerosis.

### BRIEF DESCRIPTION OF THE DRAWINGS

When read in conjunction with the drawings, the foregoing summary of the invention, as well as the following detailed description of embodiments, will be better understood. To illustrate the present disclosure, the drawings show some but not all alternative embodiments. It should be understood, however, that the disclosure is not limited to the precise arrangements and instrumentalities shown. These drawings, which are incorporated in and constitute a part of this specification, serve to explain the principles of the present disclosure.
FIG. 1 shows the infra-red spectrogram of sodium hyaluronate (HA) and plaque-targeted hyaluronic acid targeting materials (HPD).
FIG. 2 shows the cryo-electron micrographs (A, B and C) and hydrated particle size results (D, E and F) of nanoliposomes LP/R_{A90}, LP/R_{A120} and LP/R_{A150} that actively encapsulate rosuvastatin by an ethanol injection method.
FIG. 3 shows the cryo-electron micrographs (A, B and C) and hydrated particle size results (D, E and F) of nanoliposomes LP/R_{B90}, LP/R_{B120} and LP/R_{B150} that actively encapsulate rosuvastatin by hybrid hydration.
FIG. 4 shows the cryo-electron micrographs (A, B and C) and hydrated particle size results (D, E and F) of nanoliposomes HA_{H}-LP/R_{A150}, HA_{M}-LP/R_{A150} and HA_{L}-LP/R_{A150} that actively encapsulate rosuvastatin.
FIG. 5 shows the cryo-electron micrograph (A) and hydrated particle size result (B) of the targeted liposome nanocarrier delivery system HA@LP/R_{C} that actively encapsulates rosuvastatin.
FIG. 6 shows the cryo-electron micrograph and hydrated particle size (Dynamic Light Scattering, DLS) of the targeted liposome HA-LP/A_{C} that actively encapsulates atorvastatin.
FIG. 7 shows the cryo-electron micrograph and DLS of the targeted drug liposome HA-LP/Asp_{C} that actively encapsulates aspirin.
FIG. 8 shows the cryo-electron micrograph and DLS of the targeted liposome HA-LP/P_{C} that actively encapsulates pitavastatin.
FIG. 9 shows the cryo-electron micrograph and DLS of the targeted liposome HA-LP/L_{C} that actively encapsulates lovastatin.
FIG. 10 shows the cryo-electron micrograph and DLS of the targeted liposome HA-LP/Sc that actively encapsulates simvastatin.
FIG. 11 shows the cryo-electron micrograph and DLS of the targeted liposome HA-LP/P_{C} that actively encapsulates pravastatin.
FIG. 12 shows the cryo-electron micrograph and DLS of the targeted liposome HA-LP/B_{C} that actively encapsulates bezafibrate.
FIG. 13 shows the cryo-electron micrograph and DLS of the targeted liposome HA-LP/Cc that actively encapsulates ciprofibrate.
FIG. 14 shows the cryo-electron micrograph and DLS of the targeted liposome HA-LP/Am_{C} that actively encapsulates acemetacin.
FIG. 15 shows the cryo-electron micrograph and DLS of the targeted liposome HA-LP/Oc that actively encapsulates ozagrel.
FIG. 16 shows the cryo-electron micrograph and DLS of the targeted liposome HA-LP/T_{C} that actively encapsulates tirofiban.
FIG. 17 shows the cryo-electron micrograph and DLS of the targeted liposome HA@LP/R_{d} that actively encapsulates rosuvastatin.
FIG. 18 shows the cryo-electron micrograph and DLS of the targeted liposome HA@LP/A_{d} that actively encapsulates atorvastatin.
FIG. 19 shows the cryo-electron micrograph and DLS of the targeted liposome HA@LP/C_{d} that actively encapsulates ciprofibrate.
FIG. 20 shows the cryo-electron micrograph and DLS of the targeted liposome HA-LP/B_{d} that actively encapsulates bezafibrate.
FIG. 21 shows the results of investigation of long-term stability of the nanodelivery systems of the present disclosure.
FIG. 22 shows the percentages of plaque volume progression of the HA-LP/R nano drug delivery system of the present disclosure and each control group.
FIG. 23 shows a comparison of the degree of enrichment of different therapeutic drugs in the aorta.
FIGs. 24a and 24b show comparisons of the drug efficacy of different HA/lipid material mass ratios.
FIG. 25 shows a comparison of fluorescence values of cell lysates in the endocytosis assay.
FIG. 26a shows the fluorescence micrograph of the live cell assay for the LP/R_{C} liposome in the endocytosis assay; FIG. 26b shows the fluorescence micrograph of the live cell assay for the HA@LP/R_{C} nanocarrier delivery system in the endocytosis assay, wherein the blue is the nuclei stained with Hoechst33342, and the red is Cy5.5-labeled liposomes in the cell.
FIG. 27 shows the results of *in vivo* distribution and metabolism of fluorescently labeled HA@LP/R_{C} and LP/R_{C} under different time conditions.
FIG. 28 shows the change in the body weight of animals after administration of HA@LP/R_{C}.
FIG. 29 shows the reference standard curve for HPD measurement.

### DETAILED DESCRIPTION OF EMBODIMENTS

The present disclosure will be further described below by specific examples, but it should be understood that these examples are only for the purpose of more detailed description and should not be construed as limiting the present disclosure in any form.

The section describes the materials and test methods used in the test of the present disclosure. It is clear to a person skilled in the art that the materials and operation methods used in the present disclosure are well known in the art in the context, unless otherwise specified.

The reagents and instruments used in the following examples are as follows:
reagents:
DSPC, DSPE, cholesterol and DSPE-PEG2000, purchased from AVT Company;
chloroform, anhydrous ethanol, sodium bicarbonate, EDC, NHS, calcium acetate and rosuvastatin calcium, purchased from J&K Scientific;
HA, purchased from Bloomage Biotech; DSPE-PEG2000-NH₂, purchased from Sigma;
an ultrafiltration membrane pack, purchased from Merck; polycarbonate membrane, purchased from Whatman;
atorvastatin, purchased from Lunarsun;
aspirin, pitavastatin, lovastatin, simvastatin, pravastatin, bezafibrate, ciprofibrate, acemetacin, ozagrel, and tirofiban, purchased from J&K Scientific; 2-hydroxypropyl-β-cyclodextrin, purchased from Sigma-Aldrich;
instruments:
   an extruder (ATS, AE001);
   a laser particle size analyzer, purchased from Malvern Company (model: Nano-ZS).
   HPLC, purchased from Waters Company (model: e2695).

### EXAMPLE 1 Synthesis of plaque-targeted hyaluronic acid-based targeting materials (HPD)

In this example, sodium hyaluronate (HA) with an average molecular weight of 12,000 to 16,000 and DSPE-PEG2000-NH2 (DPN) were used as raw materials, and condensed by amide bonds under the action of EDC and NHSS. After crystallization and washing with ethanol, finished products of CD44-targetd targeting materials (HPD) were obtained.

The specific method was as follows. 75 g of HA was weighed and dissolved with purified water or water for injection with stirring. DPN or a solution of DPN in DMF was added, and the mass ratio of added DPN to HA was controlled to 1:1 or greater. EDC or EDC and NHSS were added, mixed well, and stirred at 25-40°C for ≥10 hours. After the completion of the reaction, an equal volume or more of anhydrous ethanol was added to the reaction solution, stirred evenly, and then left standing for 20 minutes or more. After standing, the solution was centrifuged to collect the precipitates, or the solution was concentrated through a membrane pack, and then centrifuged to collect the precipitates. The precipitates were washed by resuspending with ethanol and centrifuged, and the washing was repeated twice or more. The centrifuged precipitates obtained after washing were vacuum-dried or freeze-dried to control the moisture content of the final products to less than 10%.

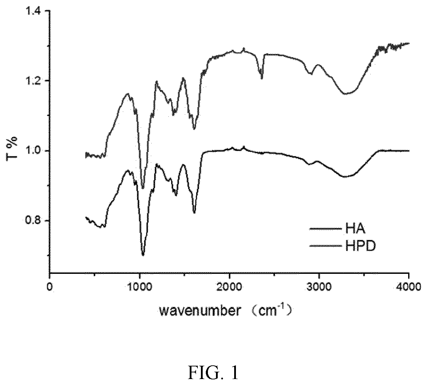

FIG. 1 shows the infra-red spectrogram of HA and HPD.

### EXAMPLE 2 Preparation of nanoliposomes actively encapsulating rosuvastatin by an ethanol injection method

In this example, nanoliposomes loaded with rosuvastatin were prepared by ethanol injection & active encapsulation method.

Distearoylphosphatidylcholine (DSPC), cholesterol and DSPE-PEG2000 (in a mass ratio of 3:1:1 or 3:1:0.5) were weighed, and the above lipid materials were dissolved with ethanol. A calcium acetate aqueous solution (150 mM or higher) was added and the ethanol phase was injected into the aqueous phase calcium acetate solution while maintaining the temperature at 40-60°C and stirring to fully hydrate the lipid material solution to form a crude liposome suspension (the volume ratio of the calcium acetate aqueous phase to the ethanol phase solution was greater than 2). Extrusion was performed three times or more through polycarbonate membranes of 600 nm, 400 nm, 200 nm and 100 nm respectively or multilayer membranes composed thereof using an extruder. The extruded nanoliposome solution was diluted 20 times or more with purified water, and nanoparticles (LP/R_{A90}, LP/R_{A120} and LP/R_{A150}) with three different sizes of particle diameters of about 90 nm, 120 nm and 150 nm were obtained by controlling the membrane pore size, with a PDI of less than 0.2 for all the nanoparticles. A dialysis bag or an ultrafiltration membrane pack was used to separate unencapsulated calcium acetate by dialysis. The inner solution was collected and mixed well with a rosuvastatin calcium solution or a mixed solution of rosuvastatin calcium and sucrose (the mass ratio of rosuvastatin calcium to the total lipid materials was less than 0.25), and incubation was performed at 4-66°C for 15 minutes or more to obtain nanoliposomes that actively encapsulated rosuvastatin calcium. The encapsulation rate of the API in the prepared nanoliposomes was greater than 80%, and the concentration of the API in the final formulation was less than or equal to 20 mg/mL.

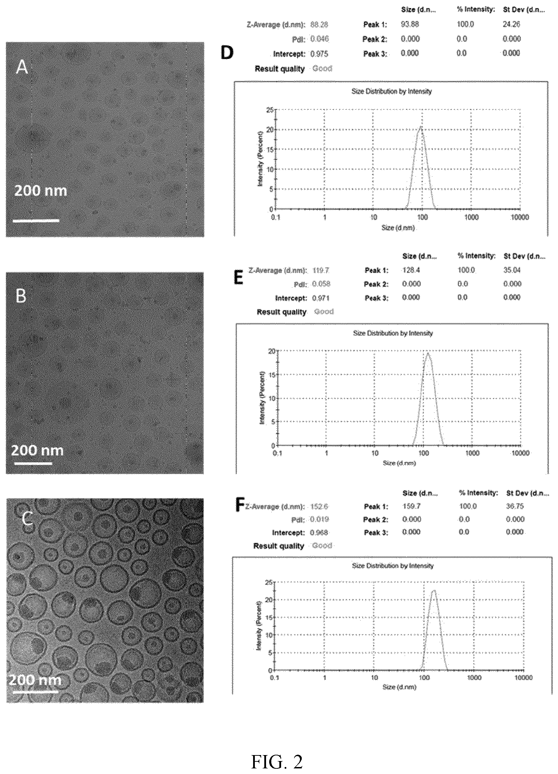

FIG. 2 shows the cryo-electron micrographs (A, B and C) and hydrated particle size results (D, E and F) of the nanoliposomes LP/R_{A90}, LP/R_{A120} and LP/R_{A150} actively encapsulating rosuvastatin by the ethanol injection method in this example.

### EXAMPLE 3 Preparation of nanoliposomes actively encapsulating rosuvastatin by hybrid hydration

In this example, nanoliposomes loaded with rosuvastatin were prepared by hybrid hydration & active encapsulation method. DSPC, cholesterol and DSPE-PEG2000 (in a weight ratio of 3:1:1-0.5) were weighed, and the above lipid materials were dissolved with ethanol. A calcium acetate aqueous solution (150 mM or higher) was added and the ethanol phase was mixed at a constant ratio with the aqueous phase using a tee pipe, a microfluidic controller or other mixers while maintaining the temperature at 40-60°C to fully hydrate the lipid materials to form a crude liposome suspension (the volume ratio of the calcium acetate aqueous phase to the ethanol phase solution was greater than 2). Extrusion was performed three times or more through polycarbonate membranes of 600 nm, 400 nm, 200 nm and 100 nm respectively or multilayer membranes composed thereof using an extruder. The extruded nanoliposome solution was diluted 20 times or more with purified water, and finally, nanoparticles (LP/R_{B90}, LP/R_{B120} and LP/R_{B150}) with three different sizes of particle diameters of about 90 nm, 120 nm and 150 nm were obtained by controlling the membrane pore size and the times of extrusion, with a PDI of less than 0.2 for all the nanoparticles. A dialysis bag or an ultrafiltration membrane pack was used to separate the unencapsulated calcium acetate by dialysis. The inner solution was collected and mixed well with a rosuvastatin calcium solution or a mixed solution of rosuvastatin calcium and sucrose (the mass ratio of rosuvastatin calcium to the total lipid materials was less than 0.25), and incubation was performed at 4-66°C for 15 minutes or more to obtain nanoliposomes that actively encapsulated rosuvastatin calcium. The encapsulation rate of the API in the prepared nanoliposomes was greater than 80%, and the concentration of the API in the final formulation was less than or equal to 20 mg/mL.

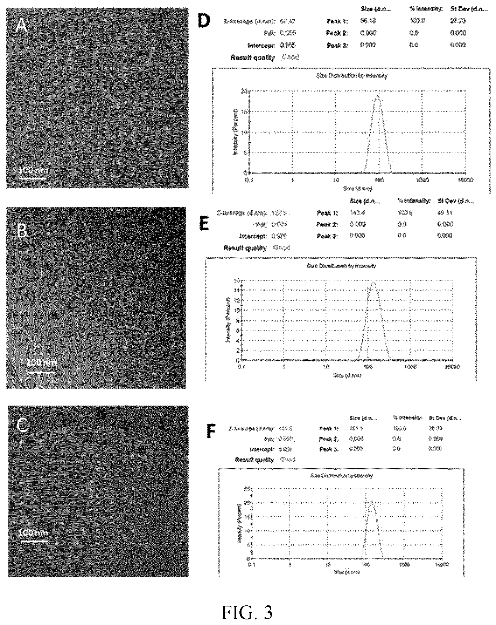

FIG. 3 shows the cryo-electron micrographs (A, B and C) and hydrated particle size results (D, E and F) of the nanoliposomes LP/R_{B90}, LP/R_{B120} and LP/R_{B150} actively encapsulating rosuvastatin by hybrid hydration in this example.

### EXAMPLE 4 Preparation of a targeted nanoliposome formulation actively encapsulating rosuvastatin

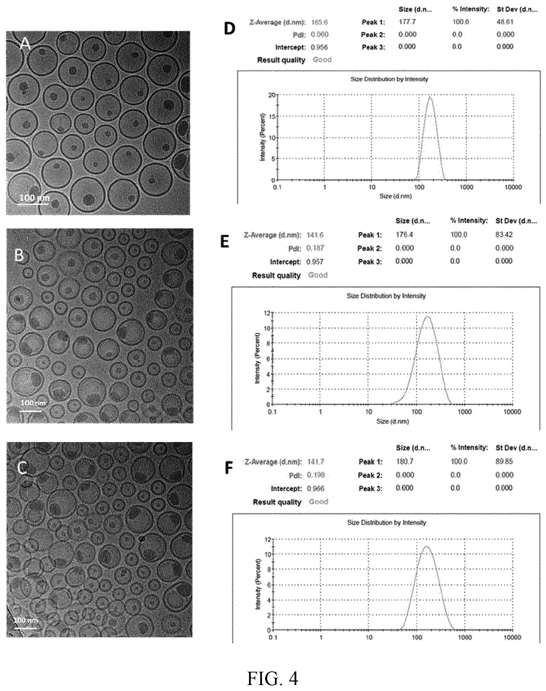

The targeting materials HPD prepared in Example 1 above were inserted into the 150 nm drug-loaded nanoliposome LP/R_{A150} prepared in Example 2 to obtain targeted drug-loaded nanoliposomes. The specific insertion method was as follows. The nanoliposome solution loaded with rosuvastatin calcium prepared in Example 2 was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain ligand-targeted liposomes (HA_{H}-LP/R_{A150}) with a high HPD concentration of 200±31 µg/mL, ligand-targeted liposomes (HA_{M}-LP/R_{A150}) with a medium HPD concentration of 100±24 µg/mL, and targeted drug-loaded liposomes (HA_{L}-LP/R_{A150}) with a low HPD concentration of 10±5 µg/mL in the final product. FIG. 4 shows the cryo-electron micrographs (A, B and C) and hydrated particle size results (D, E and F) of the nanoliposomes HA_{H}-LP/R_{A150}, HA_{M}-LP/R_{A150} and HA_{L}-LP/R_{A150} actively encapsulating rosuvastatin of this example.

The kit used a competitive enzyme-linked immunosorbent assay (ELISA). Samples, standards, HA-binding proteins (HABPs) and anti-HABP antibodies were added in sequence to coated microwells pre-coated with derivatives of hyaluronic acid (HA), and after incubation and sufficient washing, unbound components were removed. Then, HRP-labeled anti-mouse immunoglobulin G (enzyme-labeled secondary antibody) was added. After incubation and sufficient washing, unbound components were removed, and a solid phase immune complex of antigen-HA binding protein-HABP antibody-enzyme labeled antibody was formed on the solid phase surface of the microwell plate. Substrates A and B were added, and catalyzed by HRP to produce a blue product, which was finally converted to yellow under the action of a termination solution (2M sulfuric acid), and the depth of the color was negatively correlated with the hyaluronic acid (HA) in the sample. The absorbance (OD value) was measured at a wavelength of 450 nm with a microplate reader, a standard curve was fitted, and the concentration of hyaluronic acid (HA) in the sample can be calculated.

### EXAMPLE 5 Preparation of a targeted liposome nanocarrier delivery system (HA@LP/R_{C}) actively encapsulating rosuvastatin

In this example, the liposome HA@LP/R_{C} loaded with a therapeutic agent was prepared by a film dispersion method, active drug loading and chemical coupling process.

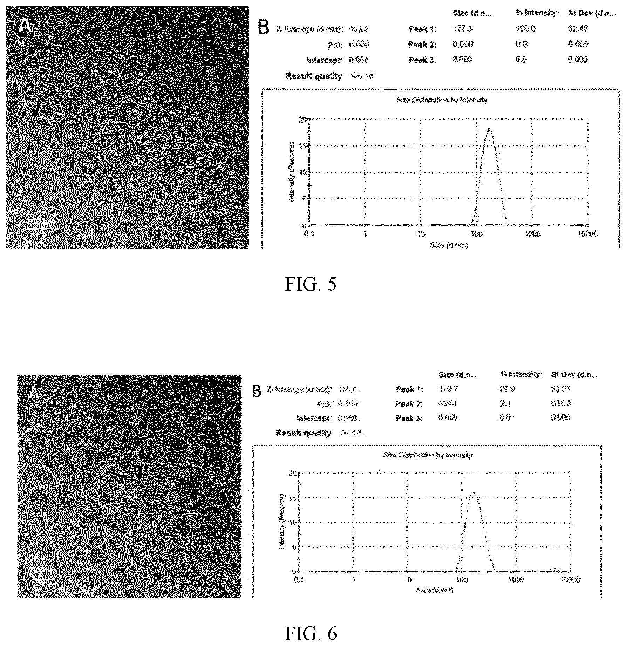

Distearoylphosphatidylcholine (DSPC), DPN, cholesterol, and polyethylene glycol-distearoylphosphatidylethanolamine (DSPE-PEG2000) (in a mass ratio of 3:0.5:1:1) were weighed and the above lipid materials were dissolved with chloroform. The organic solvent was removed by means of slow rotary evaporation (55°C water bath, 90 r/min, 30 min) to form a lipid membrane on the wall of the container. A calcium acetate aqueous solution (200 mM) was added to fully hydrate the lipid membrane in a constant-temperature water bath kettle at 45°C to form a crude liposome suspension. Extrusion was performed through polycarbonate membranes of 200 nm and 100 nm respectively using an extruder. The unencapsulated calcium acetate was separated using an ultrafiltration membrane pack. The inner solution was collected and then mixed well with 25 mL of rosuvastatin calcium aqueous solution (133 mg), and then the mixture was incubated at 65°C for 30 min to obtain drug-actively encapsulating liposomes LP/R_{C} with a particle size of 160 nm. FIG. 5 shows the particle size distribution diagram of HA@LP/R_{C} in Example 5 of the present disclosure.

The prepared nanoliposome solution loaded with rosuvastatin calcium was mixed with a pre-dissolved HA aqueous solution, and coupling agents EDC and NHSS were added. Reaction was performed while maintaining the temperature at 25°C or above for 2 hours or more. After incubation, a dialysis bag or an ultrafiltration membrane pack was used to separate the uncoupled HA by dialysis to obtain the drug-loaded targeted liposomes HA@LP/R_{C} with an HA concentration of 10-200 µg/mL in the final product.

### EXAMPLE 6 Preparation of targeted liposome nanocarriers actively encapsulating atorvastatin

In this example, the liposomes HA-LP/A_{C} loaded with a therapeutic agent was prepared by ethanol injection-active encapsulation method and an insertion method.

DSPC, cholesterol, and DSPE-PEG2000 (in a mass ratio of 3:1:1) were weighed and dissolved with ethanol. A calcium acetate aqueous solution (250 mM) was added to fully hydrate the lipid materials in a constant-temperature water bath kettle at 65°C to form a crude liposome suspension. Extrusion was performed through polycarbonate membranes of 200 nm and 100 nm respectively using an extruder to control the final particle size to 130 nm. The unencapsulated calcium acetate was separated using an ultrafiltration membrane pack. The inner solution was collected, and 1 ml of the inner solution was mixed well with 2 mL of atorvastatin calcium aqueous solution (2.5 mg), and then the mixture was incubated at 60°C for 10 min to obtain liposomes LP/A_{c} actively encapsulating drug.

The prepared nanoliposome solution loaded with atorvastatin calcium was mixed with a pre-dissolved HPD aqueous solution, and the mixture was incubated while maintaining the temperature at 25°C or above for 5 min or more. After incubation, a dialysis bag or an ultrafiltration membrane pack was used to separate the uninserted HPD by dialysis to obtain the drug-loaded targeted liposomes with an HPD concentration of 10-200 µg/mL in the final product.

FIG. 6 shows the cryo-electron micrograph and DLS of the targeted liposomes HA-LP/A_{C} actively encapsulating atorvastatin in this example.

### EXAMPLE 7 Preparation of targeted liposome nanocarriers actively encapsulating aspirin

In this example, HA-LP/Asp_{c} was prepared by ethanol injection-active encapsulation method and a post-insertion method. An aspirin aqueous solution (2 mg/mL) was dissolved by adjusting the pH with NaOH. DSPC, cholesterol and DSPE-PEG2000 (in a mass ratio of 3:1:1) were weighed, and dissolved with ethanol. A calcium acetate aqueous solution (250 mM) was added to fully hydrate the lipid material solution in a constant-temperature water bath kettle at 65°C to form a crude liposome suspension. Extrusion was performed through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of aspirin aqueous solution, and then the mixture was incubated at 40°C for 10 min to obtain liposomes LP/Asp_{C} actively encapsulating drug.

The prepared aspirin-loaded nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes with a HPD concentration of 10-200 µg/mL in the final product.

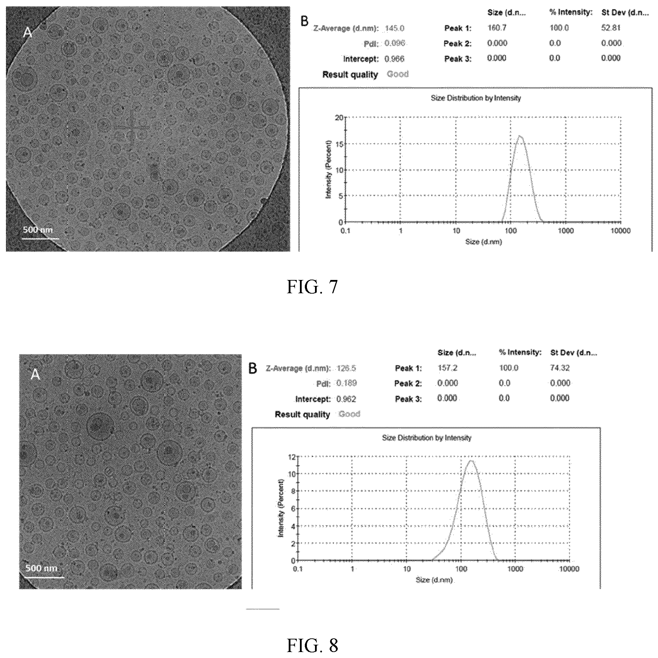

FIG. 7 shows the cryo-electron micrograph and DLS of the targeted liposomes HA-LP/Asp_{C} actively encapsulating aspirin in this example.

### EXAMPLE 8 Preparation of targeted liposome nanocarriers actively encapsulating pitavastatin

In this example, HA-LP/P_{C} was prepared by ethanol injection-active encapsulation method and a post-insertion method.

A pitavastatin aqueous solution (1 mg/mL). DSPC, cholesterol and DSPE-PEG2000 (in a mass ratio of 3:1:1) were weighed, and dissolved with ethanol. A calcium acetate aqueous solution (250 mM) was added to fully hydrate the lipid material solution in a constant-temperature water bath kettle at 65°C to form a crude liposome suspension. Extrusion was performed through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of pitavastatin aqueous solution, and then the mixture was incubated at room temperature for 10 min to obtain liposomes LP/P_{C} actively encapsulating drug.

The prepared pitavastatin-loaded nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes (HA-LP/P_{C}) with a HPD concentration of 10-200 µg/mL in the final product.

FIG. 8 shows the cryo-electron micrograph and DLS of the targeted HA-LP/P_{C} liposomes actively encapsulating pitavastatin in this example.

### EXAMPLE 9 Preparation of targeted liposome nanocarriers actively encapsulating lovastatin

In this example, HA-LP/L_{C} was prepared by ethanol injection-active encapsulation method and a post-insertion method. A lovastatin aqueous solution (Lova, 1 mg/mL) was dissolved by adjusting the pH with NaOH. DSPC, cholesterol and DSPE-PEG2000 (in a mass ratio of 3:1:1) were weighed, and dissolved with ethanol. A calcium acetate aqueous solution (250 mM) was added to fully hydrate the lipid material solution in a constant-temperature water bath kettle at 65°C to form a crude liposome suspension. Extrusion was performed 10 times through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of lovastatin aqueous solution, and then the mixture was incubated at 65°C for 10 min to obtain liposomes LP/L_{C} actively encapsulating drug.

The prepared lovastatin-loaded nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes (HA-LP/L_{C}) with a HPD concentration of 10-200 µg/mL in the final product.

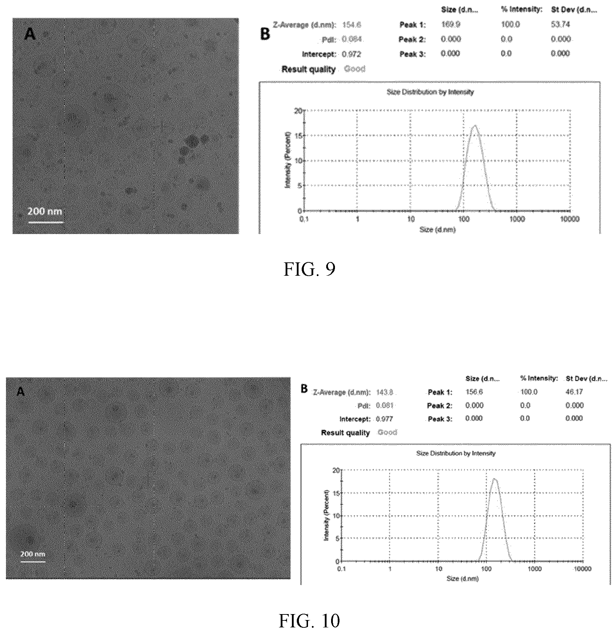

FIG. 9 shows the cryo-electron micrograph and DLS of the targeted liposomes HA-LP/L_{C} actively encapsulating lovastatin in this example.

### EXAMPLE 10 Preparation of targeted liposome nanocarriers actively encapsulating simvastatin

In this example, HA-LP/Sc was prepared by ethanol injection, active encapsulation, and post-insertion method. A simvastatin aqueous solution (Simv, 1 mg/mL) was dissolved by adjusting the pH with NaOH and adding 10% 2-hydroxypropyl-β-cyclodextrin. DSPC, cholesterol and polyethylene glycol-distearoylphosphatidylethanolamine (DSPE-PEG2000) (in a mass ratio of 3:1:1) were weighed, and dissolved with ethanol. A calcium acetate aqueous solution (250 mM) was added to fully hydrate the lipid material solution in a constant-temperature water bath kettle at 65°C to form a crude liposome suspension. Extrusion was performed 10 times through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of simvastatin aqueous solution, and then the mixture was incubated at room temperature for 30 min to obtain liposomes LP/Sc actively encapsulating drug.

The prepared simvastatin-loaded nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes (HA-LP/Sc) with a HPD concentration of 10-200 µg/mL in the final product.

FIG. 10 shows the cryo-electron micrograph and DLS of the targeted liposomes HA-LP/Sc actively encapsulating simvastatin in this example.

### EXAMPLE 11 Preparation of targeted liposome nanocarriers actively encapsulating pravastatin

In this example, HA-LP/P_{C} was prepared by ethanol injection-active encapsulation method and post-insertion method. A pravastatin aqueous solution (Pravas, 1 mg/mL). DSPC, cholesterol and polyethylene glycol-distearoylphosphatidylethanolamine (DSPE-PEG2000) (in a mass ratio of 3:1:1) were weighed, and dissolved with ethanol. A calcium acetate aqueous solution (250 mM) was added to fully hydrate the lipid material solution in a constant-temperature water bath kettle at 65°C to form a crude liposome suspension. Extrusion was performed 10 times through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of pravastatin aqueous solution, and then the mixture was incubated at 40°C for 30 min to obtain liposomes LP/P_{C} actively encapsulating drug.

The prepared pravastatin-loaded nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes (HA-LP/P_{C}) with a HPD concentration of 10-200 µg/mL in the final product.

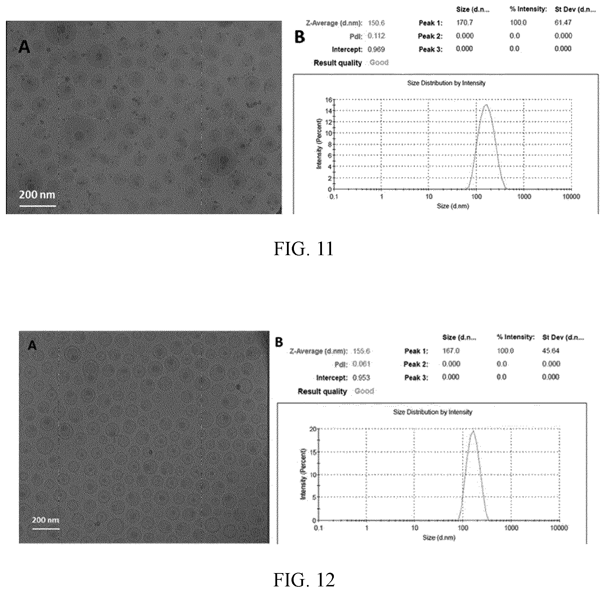

FIG. 11 shows the cryo-electron micrograph and DLS of the targeted liposomes HA-LP/P_{C} actively encapsulating pravastatin in this example.

### EXAMPLE 12 Preparation of targeted liposome nanocarriers actively encapsulating bezafibrate

In this example, HA-LP/B_{C} was prepared by ethanol injection-active encapsulation and post-insertion method. A bezafibrate aqueous solution (Beza, 1 mg/mL) was dissolved by adjusting the pH with NaOH and adding 10% 2-hydroxypropyl-β-cyclodextrin. DSPC, cholesterol and polyethylene glycol-distearoylphosphatidylethanolamine (DSPE-PEG2000) (in a mass ratio of 3:1:1) were weighed, and the lipid materials were dissolved with ethanol. A calcium acetate aqueous solution (250 mM) was added to fully hydrate the lipid material solution in a constant-temperature water bath kettle at 65°C to form a crude liposome suspension. Extrusion was performed 10 times through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of bezafibrate aqueous solution, and then the mixture was incubated at 40°C for 30 min to obtain liposomes LP/B_{C130} actively encapsulating drug.

The prepared bezafibrate-loaded nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes (HA-LP/B_{C}) with a HPD concentration of 10-200 µg/mL in the final product.

FIG. 12 shows the cryo-electron micrograph and DLS of the targeted liposomes HA-LP/B_{C} actively encapsulating bezafibrate in this example.

### EXAMPLE 13 Preparation of targeted liposome nanocarriers actively encapsulating ciprofibrate

In this example, HA-LP/Cc was prepared by ethanol injection, active encapsulation and post-insertion method. A ciprofibrate aqueous solution (Cipro, 1.5 mg/mL) was dissolved by adjusting the pH with NaOH to 6.0. Distearoylphosphatidylcholine (DSPC), cholesterol and polyethylene glycol-distearoylphosphatidylethanolamine (DSPE-PEG2000) (in a mass ratio of 3:1:1) were weighed, and the lipid materials were dissolved with ethanol. A calcium acetate aqueous solution (250 mM) was added to fully hydrate the lipid material solution in a constant-temperature water bath kettle at 65°C to form a crude liposome suspension. Extrusion was performed 10 times through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of ciprofibrate aqueous solution, and then the mixture was incubated at room temperature for 30 min to obtain liposomes LP/Cc actively encapsulating drug.

The prepared ciprofibrate nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes (HA-LP/Cc) with a HPD concentration of 10-200 µg/mL in the final product.

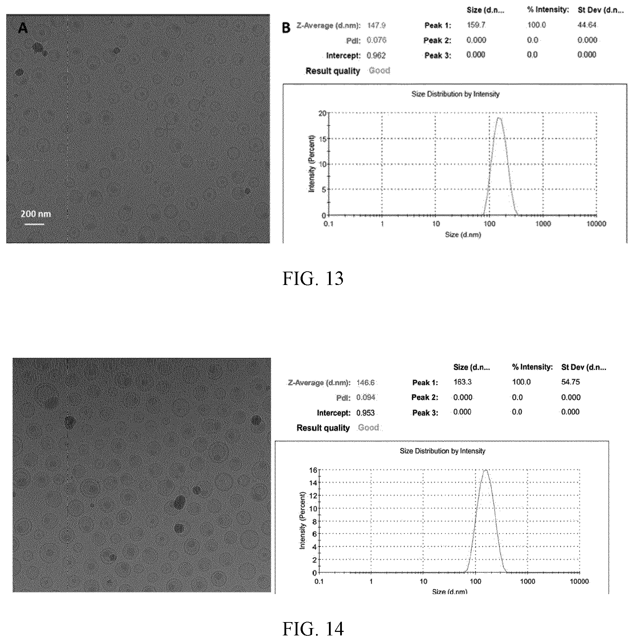

FIG. 13 shows the cryo-electron micrograph and DLS of the targeted liposomes HA-LP/Cc actively encapsulating ciprofibrate in this example.

### EXAMPLE 14 Preparation of targeted liposome nanocarriers actively encapsulating acemetacin

In this example, HA-LP/Am_{C} was prepared by ethanol injection, active encapsulation and post-insertion method. An acemetacin aqueous solution (Acemet, 1 mg/mL) was dissolved by adding 10% 2-hydroxypropyl-β-cyclodextrin. Distearoylphosphatidylcholine (DSPC), cholesterol and polyethylene glycol-distearoylphosphatidylethanolamine (DSPE-PEG2000) (in a mass ratio of 3:1:1) were weighed, and the lipid materials were dissolved with ethanol. A calcium acetate aqueous solution (250 mM) was added to fully hydrate the lipid material solution in a constant-temperature water bath kettle at 65°C to form a crude liposome suspension. Extrusion was performed 10 times through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of acemetacin aqueous solution, and then the mixture was incubated at room temperature for 30 min to obtain liposomes LP/Am_{C} actively encapsulating drug.

The prepared acemetacin nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes (HA-LP/Am_{C}) with a HPD concentration of 10-200 µg/mL in the final product.

FIG. 14 shows the cryo-electron micrograph and DLS of the targeted liposomes HA-LP/Am_{C} actively encapsulating acemetacin in this example.

### EXAMPLE 15 Preparation of targeted liposome nanocarriers actively encapsulating ozagrel

In this example, LP/Oc was prepared by ethanol injection, active encapsulation and post-insertion method. An ozagrel aqueous solution (Ozagrel, 2 mg/mL) was dissolved by adding 10% 2-hydroxypropyl-β-cyclodextrin. Distearoylphosphatidylcholine (DSPC), cholesterol and polyethylene glycol-distearoylphosphatidylethanolamine (DSPE-PEG2000) (in a mass ratio of 3:1:1) were weighed, and the lipid materials were dissolved with ethanol. A calcium acetate aqueous solution (250 mM) was added to fully hydrate the lipid material solution in a constant-temperature water bath kettle at 65°C to form a crude liposome suspension. Extrusion was performed 10 times through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of ozagrel aqueous solution, and then the mixture was incubated at room temperature for 30 min to obtain liposomes LP/Oc actively encapsulating drug.

The prepared ozagrel nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes (HA-LP/Oc) with a HPD concentration of 10-200 µg/mL in the final product.

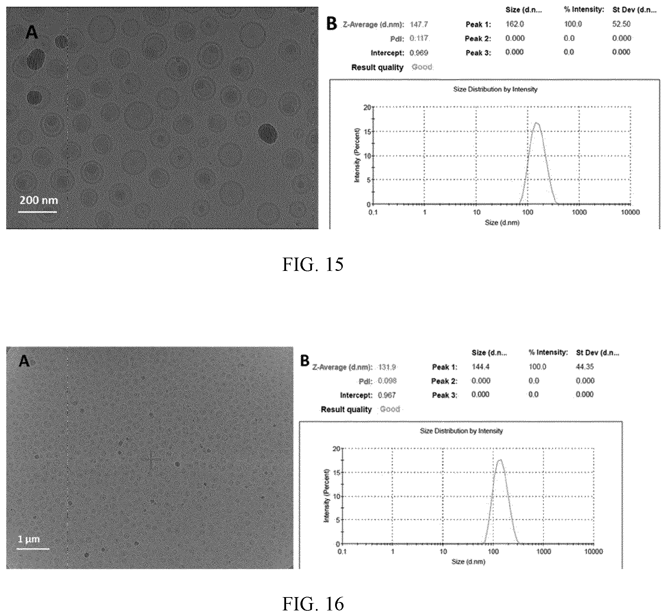

FIG. 15 shows the cryo-electron micrograph and DLS of the targeted liposomes HA-LP/Oc actively encapsulating ozagrel in this example.

### EXAMPLE 16 Preparation of targeted liposome nanocarriers actively encapsulating tirofiban

In this example, LP/T_{C} was prepared by ethanol injection, active encapsulation and post-insertion method. A tirofiban aqueous solution (Tirofiban, 2 mg/mL) was dissolved by adding 10% 2-hydroxypropyl-β-cyclodextrin. Distearoylphosphatidylcholine (DSPC), cholesterol and polyethylene glycol-distearoylphosphatidylethanolamine (DSPE-PEG2000) (in a mass ratio of 3:1:1) were weighed, and the lipid materials were dissolved with ethanol. A calcium acetate aqueous solution (250 mM) was added to fully hydrate the lipid material solution in a constant-temperature water bath kettle at 65°C to form a crude liposome suspension. Extrusion was performed 10 times through polycarbonate membranes of 200 nm and 100 nm respectively using an extruder. The unencapsulated calcium acetate was separated using an ultrafiltration membrane pack. The inner solution was collected, and 2 mL of the inner solution was mixed well with 1 mL of tirofiban aqueous solution, and then the mixture was incubated at 65°C for 30 min to obtain liposomes LP/T_{C130} actively encapsulating drug.

The prepared tirofiban nanoliposome solution was mixed with a pre-dissolved HPD aqueous solution, and the mixture was incubated while maintaining the temperature at 25°C or above for 5 minutes or more. After the incubation, a dialysis bag or an ultrafiltration membrane pack was used to separate uninserted HPD by dialysis to obtain the drug-loaded targeted liposomes (HA-LP/T_{C}) with a HPD concentration of 10-200 µg/mL in the final product.

FIG. 16 shows the cryo-electron micrograph and DLS of the targeted liposomes HA-LP/T_{C} actively encapsulating tirofiban in this example.

### EXAMPLE 17 Preparation of the targeted liposome nanocarrier delivery system (HA@LP/R_{d}) actively encapsulating rosuvastatin

Hydrogenated soybean phosphatidylcholine, DPN, cholesterol, and polyethylene glycol-distearoylphosphatidylethanolamine (DSPE-PEG2000) (in a mass ratio of 3:0.5:1:1) were weighed and the above lipid materials were dissolved with chloroform. The organic solvent was removed by means of slow rotary evaporation (55°C water bath, 90 r/min, 30 min) to form a lipid membrane on the wall of the container. A copper gluconate aqueous solution (200 mM) was added to fully hydrate the lipid membrane in a constant-temperature water bath kettle at 45°C to form a crude liposome suspension. Extrusion was performed through polycarbonate membranes of 200 nm and 100 nm respectively using an extruder. The unencapsulated calcium acetate was separated using an ultrafiltration membrane pack. The inner solution was collected and mixed well with 25 mL of rosuvastatin calcium (133 mg), and then the mixture was incubated at 65°C for 30 min to obtain liposomes LP/R_{d} actively encapsulating drug.

Coupling agents EDC and NHSS were added to an HA aqueous solution, and the resulting solution was mixed with the LP/R_{d} lipid solution to realize coupling. Reaction was performed while maintaining the temperature at 25°C or above for 2 hours or more. After completion, a dialysis bag or an ultrafiltration membrane pack was used to separate uncoupled HA by dialysis to obtain the drug-loaded targeted liposomes HA@LP/R_{d} with an HA concentration of 10-200 µg/mL in the final product.

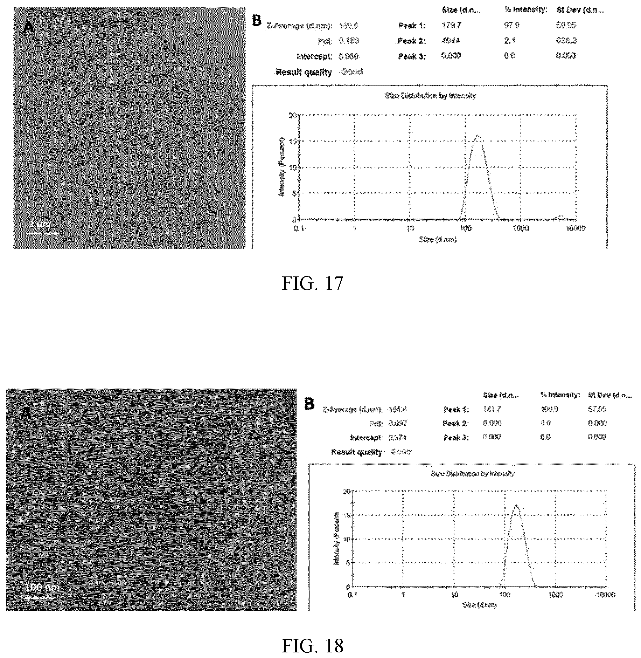

FIG. 17 shows the cryo-electron micrograph and DLS of the targeted liposomes HA@LP/R_{d} actively encapsulating rosuvastatin in this example.

### EXAMPLE 18 Preparation of targeted liposome nanocarrier delivery system (HA@LP/A_{d}) actively encapsulating atorvastatin

Dipalmitoylphosphatidylcholine, DPN, cholesterol, and polyethylene glycol-distearoylphosphatidylethanolamine (DSPE-PEG2000) (in a mass ratio of 3:0.5:1:1) were weighed and the above lipid materials were dissolved with chloroform. The organic solvent was removed by means of slow rotary evaporation (55°C water bath, 90 r/min, 30 min) to form a lipid membrane on the wall of the container. A calcium acetate aqueous solution (300 mM) was added to fully hydrate the lipid membrane in a constant-temperature water bath kettle at 45°C to form a crude liposome suspension. Extrusion was performed through polycarbonate membranes of 200 nm and 100 nm respectively using an extruder. The unencapsulated calcium acetate was separated using an ultrafiltration membrane pack. The inner solution was collected and then mixed well with 25 mL of atorvastatin calcium, and the mixture was incubated at 65°C for 30 min to obtain drug-actively encapsulating liposomes LP/A_{d} with a particle size of 130 nm.

Coupling agents EDC and NHSS were added to an HA aqueous solution, and the resulting solution was mixed with the LP/A_{d} lipid solution to realize coupling. Reaction was performed while maintaining the temperature at 25°C or above for 2 hours or more. After completion, a dialysis bag or an ultrafiltration membrane pack was used to separate uncoupled HA by dialysis to obtain the drug-loaded targeted liposomes HA@LP/A_{d} with an HA concentration of 10-200 µg/mL in the final product.

FIG. 18 shows the cryo-electron micrograph and DLS of the targeted liposomes HA@LP/A_{d} actively encapsulating atorvastatin in this example.

### EXAMPLE 19 Preparation of targeted liposome nanocarrier delivery system (HA@LP/C_{d}) actively encapsulating ciprofibrate

Sphingomyelin, cholesterol, and polyethylene glycol-distearoylphosphatidylethanolamine (DSPE-PEG2000) (in a mass ratio of 3:1:1) were weighed and the above lipid materials were dissolved with chloroform. The organic solvent was removed by means of slow rotary evaporation (55°C water bath, 90 r/min, 30 min) to form a lipid membrane on the wall of the container. A calcium bicarbonate aqueous solution (250 mM) was added to fully hydrate the lipid membrane in a constant-temperature water bath kettle at 45°C to form a crude liposome suspension. Extrusion was performed through polycarbonate membranes of 200 nm and 100 nm respectively using an extruder. The unencapsulated calcium bicarbonate was separated using an ultrafiltration membrane pack. The inner solution was collected and then mixed well with 25 mL of ciprofibrate, and the mixture was incubated at 65°C for 30 min to obtain drug-actively encapsulating liposomes LP/C_{d} with a particle size of 130 nm.

The HPD aqueous solution prepared in Example 1 was mixed and incubation was performed while maintaining the temperature at 25°C or above for 30 min or more. After the incubation, a dialysis bag or an ultrafiltration membrane pack was used to separate uninserted HPD by dialysis to obtain the drug-loaded targeted liposomes HA@LP/C_{d} with an HPD concentration of 10-200 µg/mL in the final product.

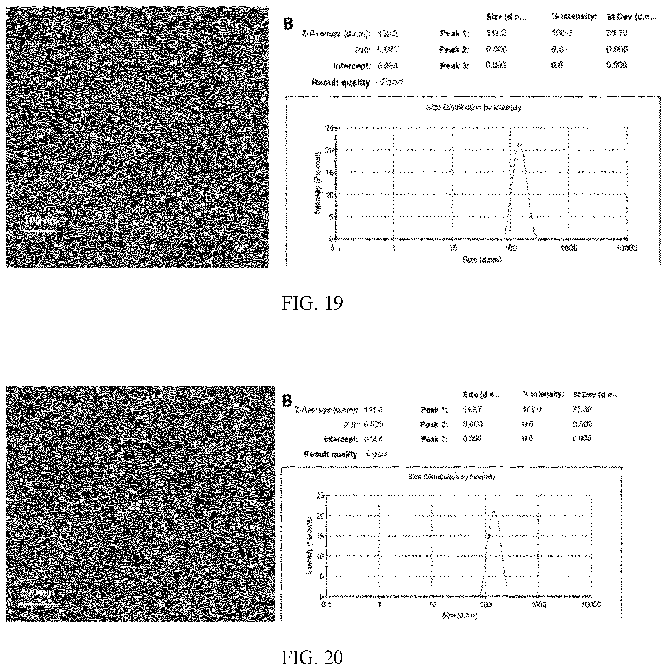

FIG. 19 shows the cryo-electron micrograph and DLS of the targeted liposomes HA@LP/C_{d} actively encapsulating ciprofibrate in this example.

### EXAMPLE 20 Preparation of targeted liposome nanocarrier delivery system (HA-LP/B_{d}) actively encapsulating bezafibrate

In this example, bezafibrate-loaded nanoliposomes were prepared by ethanol injection & active encapsulation method.

Distearoylphosphatidylserine (DOPS), cholesterol and DSPE-PEG2000 (in a mass ratio of 3:1:1) were weighted, and the above lipid materials were dissolved with ethanol. A magnesium citrate aqueous solution (200 mM) was added and the ethanol phase was injected into the magnesium citrate aqueous solution while maintaining the temperature at 40-60°C and stirring to fully hydrate the lipid material solution so as to form a crude liposome suspension (the volume ratio of the magnesium citrate aqueous phase to the ethanol phase solution was greater than 2). A dialysis bag or an ultrafiltration membrane pack was used to separate the unencapsulated magnesium citrate by dialysis. The inner solution was collected and mixed well with a bezafibrate solution (the mass ratio of bezafibrate to the total lipid materials was less than 0.25), and then incubation was performed at 4-66°C for 15 minutes or more to obtain nanoliposomes actively encapsulating bezafibrate. The prepared nanoliposomes have an encapsulation rate of greater than 20% for the API, and the final formulation has a concentration of API of less than or equal to 20 mg/mL.

The HPD aqueous solution prepared in Example 1 was mixed and incubation was performed while maintaining the temperature at 25°C or above for 30 min or more. After the incubation, a dialysis bag or an ultrafiltration membrane pack was used to separate uninserted HPD by dialysis to obtain the drug-loaded targeted liposomes HA-LP/B_{d} with an HPD concentration of 10-200 µg/mL in the final product.

FIG. 20 shows the cryo-electron micrograph and DLS of the targeted liposomes HA-LP/B_{d} actively encapsulating bezafibrate in this example.

### Assay Example 1 Stable and controllable properties of the nanocarrier delivery systems of the present disclosure

The prepared nanocarrier delivery systems loaded with the therapeutic agents, as disclosed in the present disclosure, are taken as examples to show that the delivery system of the present disclosure has stable and controllable properties and is therefore suitable for the treatment of atherosclerosis or an atherosclerosis-related disease.

### 1. Method for the determination of drug concentration

The loaded drugs rosuvastatin, tirofiban, ozagrel, acemetacin, ciprofibrate, bezafibrate, pravastatin, aspirin, pitavastatin calcium, lovastatin, and simvastatin have strong ultraviolet absorption properties, and thus by using the HPLC-UV method, a standard quantitative equation can be established with the concentration of the drug solution (X) versus the peak area of the HPLC chromatographic peak (Y).

The HPLC analysis method involved mobile phase A (1.0% trifluoroacetic acid: acetonitrile: water (1:29:70)); and mobile phase B (1.0% trifluoroacetic acid:water:acetonitrile (1:24:75)). The column temperature was 40°C and the model was Inertsil ODS-3 (150*3.0 mm). The system was equilibrated, and after the baseline was stable, the sample was injected.

### 2. Determination of hydrated particle size

The particle sizes of the delivery systems of the present disclosure were measured by a Malvern intelligent laser particle size analyzer. The results are shown in Table 1.

### 3. Determination of HPD in formulations

The kit used a competitive enzyme-linked immunosorbent assay (ELISA). Samples, standards, HA-binding proteins (HABPs) and anti-HABP antibodies were added in sequence to coated microwells pre-coated with derivatives of hyaluronic acid (HA), and after incubation and sufficient washing, unbound components were removed. Then, HRP-labeled anti-mouse immunoglobulin G (enzyme-labeled secondary antibody) was added. After incubation and sufficient washing, unbound components were removed, and a solid phase immune complex of antigen-HA binding protein-HABP antibody-enzyme labeled antibody was formed on the solid phase surface of the microwell plate. Substrates A and B were added, and catalyzed by HRP to produce a blue product, which was finally converted to yellow under the action of a termination solution (2M sulfuric acid), and the depth of the color was negatively correlated with the hyaluronic acid (HA) in the sample. The absorbance (OD value) was measured at a wavelength of 450 nm with a microplate reader, a standard curve was fitted, and the concentration of hyaluronic acid (HA) in the sample can be calculated.

The Hyaluronic Acid (HA) Quantitative Detection ELISA Kit was used for the detection. An appropriate amount of HPD was accurately weighed, and water was added to prepare a control stock solution of about 2.5 mg/mL. The control stock solution was diluted with an equal proportion of water to prepare a linear working solution (0-1.25 mg/mL). An appropriate amount of HPD-free formulation solution was taken, and mixed with each linear working solution respectively to serve as a standard linear solution. An appropriate amount of formulation solution was taken, and diluted with an HPD-free formulation solution as needed, and an appropriate amount of water was added to make the proportion of liposomes in the test formulation solution the same as that in the standard linear solution to serve as a test sample solution. The standard linear solution and the test sample solution were dissolved respectively by adding a 10% Triton solution and mixed well, and then the detection using the ELISA kit was performed. The operation was performed following the operating procedures of the ELISA kit. The absorbance (OD value) was measured at a wavelength of 450 nm with a microplate reader, a standard curve was fit with four parameters, and the concentration of HPD in the test sample was calculated.

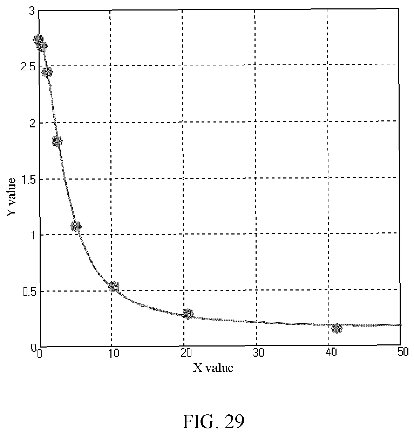

A reference standard curve diagram is shown in FIG. 29, and the fit equation is y=(A-D)/[1+(x/C)^B]+D wherein A=2.763; B=1.777; C=3.681; D=0.146; and r2=0.999.

### 4. Determination of encapsulation rate

A certain amount of nanocarriers was taken. The amounts of free drug and total drug were measured by the HPLC method, and the encapsulation rate was calculated in accordance with Equation 1 below. The results are shown in Table 1. $encapsulation rate \left(%\right) = \left(1-Mfree drug amount/Mtotal drug amount\right) * 100 %$ Equation 1

**Table 1 The brief description of the process, and the results of average particle sizes and encapsulation rate of the nanocarrier delivery systems of the preset disclosure**

| Name | Process Description | Hydrated particle size (Z-average) | Drug encapsulation rate (%) |
|---|---|---|---|
| LP/R_{A90} | Ethanol injection method and active encapsulation method | 88 | 91.7±1.8 |
| LP/R_{A120} | | 120 | 93.2±1.7 |
| LP/R_{A150} | | 152 | 95.6±1.7 |
| LP/R_{B90} | Hybrid hydration and active encapsulation method | 89 | 93.5±1.2 |
| LP/R_{B120} | | 128 | 94.4±1.4 |
| LP/R_{B150} | | 142 | 95.4±0.85 |
| HA_{H}-LP/R_{A150} | Ethanol injection method, active encapsulation method, and post-insertion of HPD | 165 | 95.5±1.24 |
| HA_{M}-LP/R_{A150} | | 142 | |
| HA_{L}-LP/R_{A150} | | 141 | |
| HA@LP/R_{C} | Film dispersion method, active drug loading, and chemical coupling | 164 | 93.5±1.5 |
| HA-LP/A_{C} | Ethanol injection-active encapsulation method and insertion method | 170 | 91.5±1.9 |
| HA-LP/Asp_{C} | Ethanol injection-active encapsulation method and post-insertion method | 145 | 92.5±1.7 |
| HA-LP/P_{C} | Ethanol injection-active encapsulation method and post-insertion method | 126 | 90.5±1.6 |
| HA-LP/L_{C} | Ethanol injection-active encapsulation method and post-insertion method | 155 | 91.5±2.2 |
| HA-LP/S_{C} | Ethanol injection-active encapsulation method and post-insertion method | 144 | 94.4±1.5 |
| HA-LP/P_{C} | Ethanol injection-active encapsulation method and post-insertion method | 150 | 95.1±0.9 |
| HA-LP/B_{C} | Ethanol injection-active encapsulation method and post-insertion method | 155 | 95.7±0.8 |
| HA-LP/C_{C} | Ethanol injection-active encapsulation method and post-insertion method | 148 | 93.4±1.4 |
| HA-LP/Am_{C} | Ethanol injection-active encapsulation method and post-insertion method | 146 | 94.7±1.1 |
| HA-LP/O_{C} | Ethanol injection-active encapsulation method and post-insertion method | 147 | 91.9±1.5 |
| HA-LP/T_{C} | Ethanol injection, active encapsulation, and post-insertion method | 132 | 92.7±1.3 |
| HA@LP/R_{d} | Film hydration, active encapsulation, chemical coupling, and copper gluconate | 169 | 91.3±0.9 |
| HA@LP/A_{d} | Film hydration, active encapsulation, chemical coupling, and calcium acetate | 165 | 94.3±1.6 |
| HA@LP/C_{d} | Film hydration, active encapsulation, post-insertion method, and calcium bicarbonate | 139 | 91.3±2.2 |
| HA-LP/B_{d} | Ethanol injection, active encapsulation method, post-insertion method, and magnesium citrate | 142 | 91.7±1.7 |

| | | | |
|---|---|---|---|
| Note: The above data are all expressed in the form of "average + standard deviation" of the results of 3 determinations in parallel. | | | |

### 5. Investigation of long-term stability

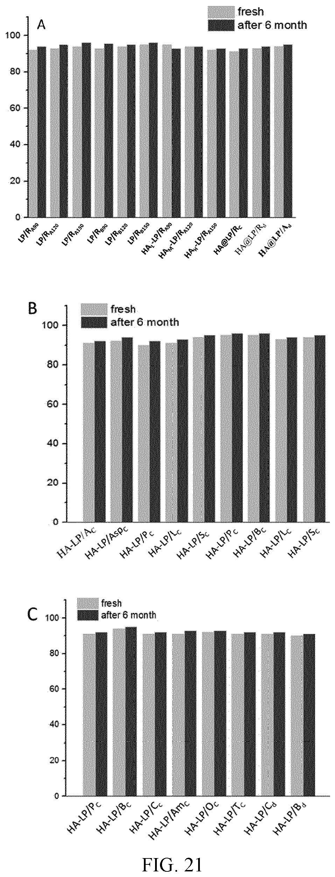

The nanocarrier delivery systems of the present disclosure were stored at 4°C, and sampled at different time points. The changes in the hydrated particle sizes thereof were detected by a laser particle analyzer. The HPLC method was used to detect the changes in the content and the encapsulation rate thereof. The results are shown in FIG. 21.

### Assay Example 2 Verification of the targeting ability of the targeted liposome nanocarrier delivery systems of the present disclosure

The purpose of this assay example is to verify the ability of the targeted liposome nano drug carrier delivery systems of the present disclosure, such as the HA@LP/R_{C} carrier delivery system, to target atherosclerotic plaques. Specifically, since atherosclerotic plaques are rich in macrophages which highly express CD44 receptors, in this study, THP1 macrophages were selected, activated with lipopolysaccharide, and incubated together with fluorescence labeled targeted HA@LP/R_{C} or non-targeted LP/R_{C}, followed by live cell microscopic observation and fluorescence value measurement. The targeting ability of the HA-targeted liposomes to target activated macrophages that highly express CD44 receptors was assessed by comparing the results of the target head HA-containing liposome group with the non-targeted liposome group.

### 1. Assay reagents and instruments

**Table 2 List of reagents used in the assay**

| **Name** | **Manufacturer** | **Model** |
|---|---|---|
| Phorbol 12-myristate 13-acetate (PMA) | Sigma | P1585 |
| THP1 human macrophage | Wuhan Procell Life Science & Technology Co., Ltd. | CL-0233 |
| Lipopolysaccharide (LPS) | Fisher Scientific | 00-4976-93 |
| Hoechst33342 | BD Biosciences | 561908 |
| RPMI1640 | Fisher Scientific | 11998075 |
| Gibco Fetal Bovine Serum | Fisher Scientific | 11573397 |
| RIPA cell lysate | Solarbio Life Sciences | R0010-20ml |
| Cy5.5 fluorescent dyes | Xi'an Ruixi | 210892-23-2 |
| Ultrafiltration tube (MWCD 50KD) | Merck millipore | UFC910096 |

**Table 3 List of instruments used in the assay**

| **Name** | **Manufacturer** | **Model** |
|---|---|---|
| Fluorescent plate reader | Perkin Elmer | EnVision2105 |
| Intelligent living cell detection system | BioTek | Lionheart FX |
| Pharmaceutical low speed horizontal rotor centrifuge | Beijing Baiyang | 52A |

### 2. Assay procedure

### 2.1 Fluorescent labeling of the test sample

1.0 mg of Cy5.5 dye was accurately weighed and dissolved in 1 ml of anhydrous ethanol to prepare a 1 mg/ml Cy5.5 solution. 5 ml of targeted HA@LP/R_{C} or non-targeted LP/R_{C} liposomes were taken respectively, and 0.1 mL of 1mg/ml Cy5.5 solution was added. The mixture was stirred at 150 rpm for 12 h, and fluorescent labeling was performed. Then, filtration was performed with an ultrafiltration tube to remove unreacted fluorescent molecules. Fluorescent detection was performed on the fluorescently labeled liposomes obtained after filtration using a fluorescent microplate reader. The detected fluorescently labeled liposomes were used for the subsequent endocytosis assay.

### 2.2 Endocytosis assay

The operating procedure for cell resuscitation of this platform was followed to carry out the operation steps. The conventional THP1 cell culture medium (RPMI1640+10% FBS) of this platform was used as the medium. After resuscitation, the cells were resuspended at a density of 5×10⁵/ml and seeded in a T75 culture flask. 10 ml of THP1 cells grown in suspension were transferred to a 15 ml centrifuge tube, centrifuged at 1000 rpm for 5 min, resuspended in 1 ml of culture medium and counted to prepare a cell suspension with a concentration of 1×10⁵/200 µl. PMA was added to 1×10⁵/200 µl cell suspension to a final concentration of 100 ng/ml. After mixed evenly, the cell suspension was seeded in a 96-well plate (200 µl/well) and stimulated continuously for 72 hours. After 72 hours of stimulation, the original medium was discarded, and a fresh medium containing lipopolysaccharide (LPS) (the LPS solution was diluted 1,000 times in the fresh medium to a final concentration of 2.5 µg/mL) was added. LPS treatment was performed for 2 hours.

Two groups were set up in the assay and the addition was conducted separately. 1 ml of fluorescently labeled liposomes were taken and diluted 10 times by volume in the fresh culture medium. At that time, the concentration of rosuvastatin was 0.2 mg/ml. The culture solution containing LPS was discarded from the 96-well plate, and the diluted fluorescently labeled test sample was added; the process was repeated three times for each group, and incubation was continued at 37°C for 30 min.

The plate was washed once with PBS, and 2 µl of Hoechst33342 mother liquor was diluted in 10 ml of fresh medium to prepare a diluted Hoechst33342 solution. The diluted Hoechst33342 solution was incubated with the cells for 10 minutes to stain the nucleus. Photographs were taken by the Lionheart FX intelligent live cell imaging analysis system. After taking photos, the staining solution was discarded from the well plate, 100 µl of RIPA cell lysate was added, and the fluorescence intensity in the well plate was measured by a fluorescence plate reader. From the assay data obtained, the average and standard deviation were calculated using Graph Pad Prism 8 software, and statistical analysis of t test was performed, wherein a p value of <0.05 was considered statistically significant.

### 3. Assay results

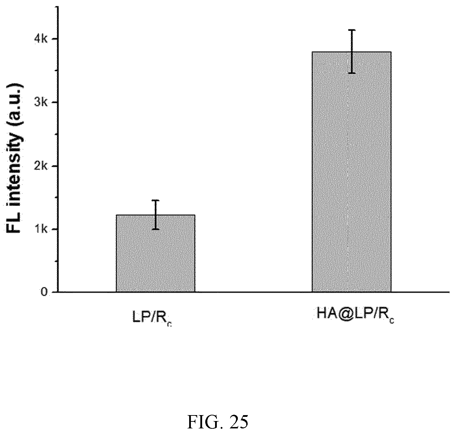

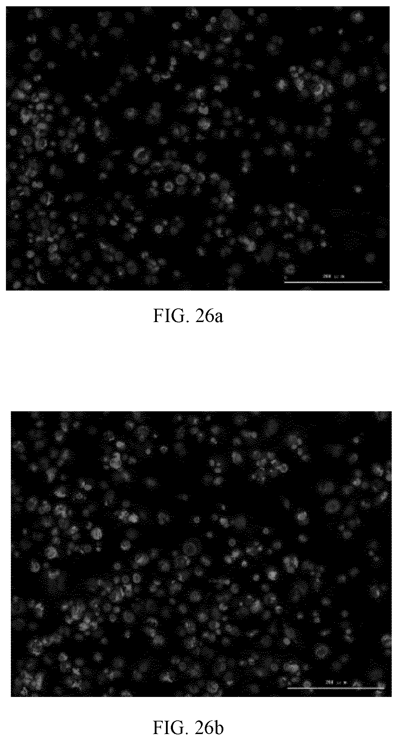

In the endocytosis assay, the endocytosis efficiency of THP1 cells stimulated by LPS for HA@LP/R_{C} and LP/R_{C} was compared. The assay results are shown in FIG. 25. Compared with the non-targeted LP/R_{C} liposome group, the fluorescence value of the HA@LP/R_{C} group increased significantly, and was 3 times that of the non-targeted liposome group. Fluorescence micrograph observation also shows (see FIG. 26) that the intracellular red fluorescence in the HA@LP/R_{C} group significantly increased compared with that in the non-targeted LP/R_{C} group. This demonstrates that the endocytosis efficiency of the THP1 cells stimulated by LPS for HA@LP/R_{C} was significantly higher than that for non-targeted liposomes.

The results show that there were significantly more cells showing red fluorescence in the targeted group than in the non-targeted liposome group. In addition, compared with the non-targeted liposome group, the fluorescence value of the targeted group also increased significantly. It can be concluded that liposomes containing HA as a target head can be specifically taken up by activated macrophages that highly express CD44 receptors.

### Assay Example 3 Research on the in vivo distribution and metabolism of the targeted liposome nanocarrier delivery systems of the present disclosure

The purpose of this assay example is to preliminarily reveal the distribution and metabolism of the targeted liposome nano drug carrier delivery systems of the present disclosure in animals. In this assay, the HA@LP/R_{C} and LP/R_{C} liposomes used in the endocytosis assay in Assay Example 2 continued to be used in the preliminary study of the distribution and metabolism in animals.

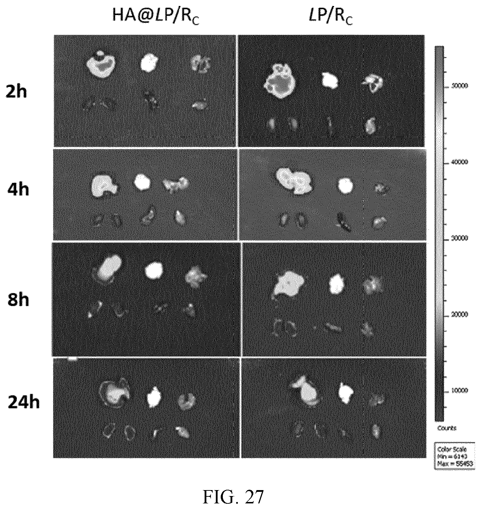

Specifically, 6-week-old ApoE-/- mice were fed a high-fat diet for 28 weeks, and aortic plaques were generated to form AS animal models. After intravenous administration of the targeted nano statin formulation (HA@LP/R_{C} at a dose of 5 mg/kg) and non-targeted nano-formulation, the tissue distribution and metabolism in the model animals were studied. As shown in FIG. 27, the nano-formulations were mainly enriched in the liver *in vivo,* and cannot cross the blood-brain barrier, and there was no significant difference in the *in vivo* distribution and metabolism of the targeted and non-targeted drugs.

Furthermore, the assay animals were randomly divided into the following groups, each including 6 animals:
rosuvastatin oral administration group: treatment was performed by oral administration at a dose of 0.5 mg rosuvastatin/kg body weight; and
rosuvastatin intravenous injection group: treatment was performed by intravenous administration at a dose of 0.5 mg rosuvastatin HA@LP/R_{C}/kg body weight;
the treatment group was treated once every other day for a total of 11 times of administration.

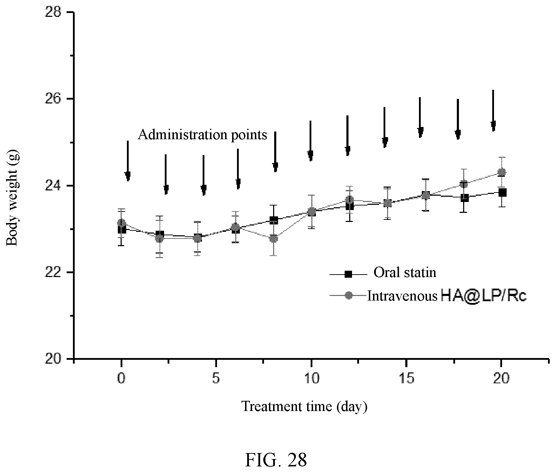

The curve of animal body weight change with treatment time is shown in FIG. 28. It can be seen from FIG. 28 that the animal body weight increased during the period of treatment, and no death was observed, indicating that the drug safety is good.

### Assay Example 4 In vivo assay of the effect of targeted liposome nanocarrier delivery system of the present disclosure on atherosclerosis

The purpose of this test example is to verify the *in vivo* therapeutic effect of the targeted liposome nano drug carrier delivery systems of the present disclosure, such as the HA_{M}-LP/R_{A150} carrier delivery system, on arterial plaques.
1. Establishment of animal models and grouping of assay animals
   (1) A normal saline solution of free rosuvastatin was prepared, and a nanodelivery system loaded with the therapeutic agent was prepared by the method described in the above Example 2.
   (2) Establishment of ApoE-/- mouse models of atherosclerosis:
      SPF-grade ApoE-/- mice (42 mice, 5-6 weeks old, weight 20±1 g) were taken as assay animals. The mice were fed an adaptive high-fat diet (fat 10% (w/w), cholesterol 2% (w/w), sodium cholate 0.5% (w/w), and the rest being normal feed for mice) for 4 weeks, and then anaesthetized via intraperitoneal injection of 1% sodium pentobarbital (prepared by adding 1 mg of sodium pentobarbital to 100 ml of normal saline) at a dose of 40 mg/kg. Then, the mice were fixed on the surgical plate in the supine position, and disinfected around the neck with 75% (v/v) alcohol. The neck skin was cut longitudinally, and the anterior cervical gland was bluntly separated, and the beating left common carotid artery can be observed on the left side of the trachea. The common carotid artery was carefully separated to the bifurcation. A silicone cannula with a length of 2.5 mm and an inner size of 0.3 mm was placed on the outer periphery of the left common carotid artery. The proximal and distal segments of the cannula were narrowed and fixed by filaments. Local tightening caused turbulent blood flow in the proximal end with increased shear force, causing damage to the intima of the blood vessel. The carotid artery was repositioned and the anterior neck skin was intermittently sutured. All operations were performed under a 10× stereomicrograph. After awakened from the surgery, the mice were returned to the cage, where the ambient temperature was maintained at 20-25°C, and the light was kept under a 12 h/12 h light/dark cycle. At the 4th week after the surgery, lipopolysaccharide (LPS) (1 mg/kg in 0.2 ml phosphate buffered saline, Sigma, U.S.A.) was injected intraperitoneally twice a week for 10 weeks to induce chronic inflammation. At the 8th week after the surgery, mice were placed in a 50 ml syringe (sufficient vents reserved) to trigger restrictive mental stress, 6 hours/day, 5 days per week for a total of 6 weeks. The mouse model of atherosclerosis was completed at the 14th week after the surgery.
   (3) Grouping and treatment of assay animals:
      The assay animals were randomly divided into the following groups, 6 mice in each group:
      control group of atherosclerosis model: this group of animals do not undergo any therapeutic treatment;
      rosuvastatin oral administration group: treatment was performed by oral administration at a dose of 0.66 mg rosuvastatin per kg body weight;
      rosuvastatin intravenous administration group: treatment was performed by intravenous administration at a dose of 0.66 mg rosuvastatin per kg body weight;
      nano-formulation (HA_{M}-LP/R_{A150}) administration group: treatment was performed by intravenous administration at a dose of 0.66 mg rosuvastatin per kg body weight;
      except for the control group of atherosclerosis model, the treatment group was treated once every other day for a total of 4 weeks of administration. For animals in each group, carotid MRI scans were performed before and after treatment weekly to detect plaque and lumen area, and the percentage of plaque volume progression was calculated based on the scanned thickness.

Percentage of plaque volume progression=(plaque volume after treatment-plaque volume before treatment)/plaque volume before treatment.

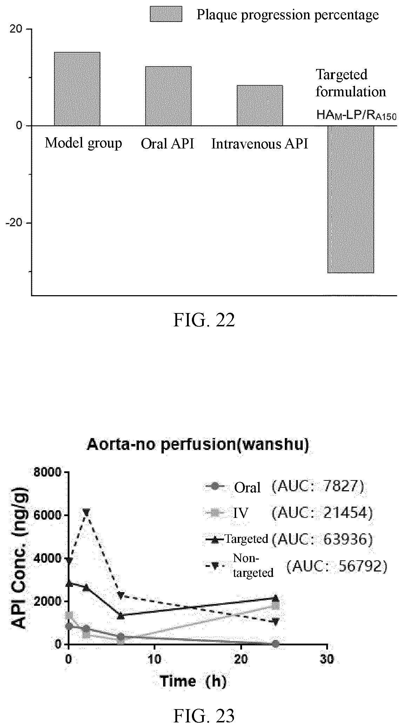

FIGs. 22 and 23 display the *in vivo* therapeutic effect of the carrier delivery system HA_{M}-LP/R_{A150} of the present disclosure on atherosclerosis. As shown in FIG. 22, oral and intravenous administration of free statin can merely delay plaque progression, but cannot lead to regression of existing plaques. However, targeted nano drug-loaded therapy not only significantly inhibited the progress of plaque, but also led to a significant reversal and regression of plaque volume by up to 30.3%. As shown in FIG. 23, compared with oral administration and injection of statins, the nanocarrier delivery system showed its extremely significant function of aortic enrichment. Compared with the non-targeted delivery system (LP/R_{A150}), this enrichment effect became more obvious under the effect of targeted nano-formulation (HA_{M}-LP/R_{A150}). To sum up, as for atherosclerosis in mice, free rosuvastatin cannot exhibit the therapeutic effect of reversing plaques, whether given by oral administration or intravenous administration. However, when statin is loaded into the nanocarrier delivery system of the present disclosure, the therapeutic effect on atherosclerosis is significantly improved, and the therapeutic effect of narrowing plaque is achieved, and the nano system with functional modification has better effect.

### 2. Comparison of the drug efficacy of different HA/lipid material mass ratios

6-week-old ApoE-/- mice were fed a high-fat diet for 28 weeks, and aortic plaques were generated to form an animal model of AS. According to blood lipid and body weight, the successfully modeled animals were randomly divided into a control group of plaque model, non-targeted nano statin formulation group (LP/R_{A150}, 5 mg/kg) and targeted nano statin formulation-1 (HA_{L}-LP/R_{A150}, at a dose of 5 mg/kg), targeted nano statin formulation-2 (HA_{M}-LP/R_{A150}, at a dose of 5 mg/kg, iv), and targeted nano statin formulation-3 (HA_{H}-LP/R_{A150}, at a dose of 5 mg/kg, iv), 10 mice in each group. The treatment was performed once a day for a total of 28 days, and the course of treatment was 4 weeks. After the course of treatment, macropathology (en-face) of aortic plaques was performed. The targeted formulations could reverse the aortic plaques of AS model mice in a short time, and the HA_{M-}LP/R_{A150} and HA_{L}-LP/R_{A150} formulation groups showed nearly significant plaque reversal.

The above studies show that in order to develop ligand-coupled active targeting nano-delivery systems, attention needs to be paid to the coupling density of the ligand on the surface of nanocarriers. For ligand molecules with special physiological functions, neither low density nor excessive coupling can lead to good targeting effects, suggesting that in the development of such products there is a particular need to achieve a controllable coupling process.

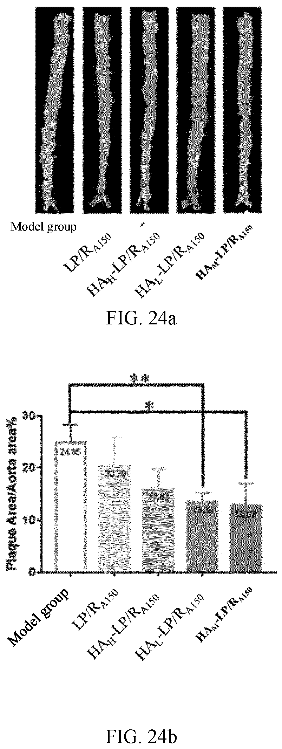

FIG. 24a and FIG. 24b show comparisons of the drug efficacy of different mass ratios of HA to lipid materials.

## Claims

1. An actively encapsulating liposome nanocarrier delivery system, **characterized in that** the liposome nanocarrier delivery system includes a liposome carrier and a substance used for preventing and/or treating atherosclerosis or an atherosclerosis-related disease actively encapsulated by the liposome carrier;
Wherein:
- materials of the liposome carrier comprise phospholipid and cholesterol;
- the liposome nanocarrier delivery system is modified with a targeting ligand which is hyaluronic acid and DSPE-PEG2000-NH₂ coupled through an amide bond; and
- in the liposome nanocarrier delivery system, the final concentration of the targeting ligand is 10-200 µg/mL.

2. The liposome nanocarrier delivery system according to claim 1, **characterized in that**, in the liposome nanocarrier delivery system, the final concentration of the targeting ligand is 10-100 µg/mL.

3. The liposome nanocarrier delivery system according to claim 1 or 2, **characterized in that** the phospholipid is one or more selected from the following: hydrogenated soybean phosphatidylcholine, distearoylphosphatidylcholine, distearoylphosphatidylethanolamine, polyethylene glycol-distearoylphosphatidylethanolamine, distearoylphosphatidylglycerol, distearoylphosphatidylserine, dioleoylphosphatidylcholine, dioleoylphosphatidylethanolamine, dioleoylphosphatidylserine, dioleoylphosphatidylethanolamine-polyethylene glycol, dicinnamoylphosphatidylcholine, dicinnamoylphosphatidylethanolamine, dicinnamoylphosphatidylserine, dicinnamoylphosphatidylethanolamine-polyethylene glycol, dipalmitoylphosphatidylcholine, dipalmitoylphosphatidylethanolamine, dipalmitoylphosphatidylserine, dipalmitoylphosphatidylethanolamine-polyethylene glycol, dierucoylphosphatidylcholine, dierucoylphosphatidylethanolamine, dierucylphosphatidylserine, dierucylphosphatidylethanolamine-polyethylene glycol, sphingomyelin, egg phosphatidylcholine, egg phosphatidylethanolamine, and egg phosphatidylglycerol.

4. The liposome nanocarrier delivery system according to any one of claims 1-3, **characterized in that** the mass ratio of the substance used for preventing and/or treating atherosclerosis or an atherosclerosis-related disease to the liposome carrier is 1:0.1 to 50, preferably 1:0.2 to 30, yet preferably 1:0.5 to 20.

5. The liposome nanocarrier delivery system according to any one of claims 1-4, **characterized in that** the substance used for preventing and/or treating atherosclerosis or an atherosclerosis-related disease is a weakly acidic substance;
yet preferably, the substance used for preventing and/or treating atherosclerosis or an atherosclerosis-related disease is one or more selected from the following: statins, fibrates, antiplatelet drugs, PCSK9 inhibitors, anticoagulant drugs, angiotensin converting enzyme inhibitors, calcium ion antagonists, MMPs inhibitors, β-receptor blockers, glucocorticoid, and pharmaceutically acceptable salts thereof;
yet preferably, the substance used for preventing and/or treating atherosclerosis or an atherosclerosis-related disease is one or more selected from the following: lovastatin, atorvastatin, rosuvastatin, simvastatin, fluvastatin, pitavastatin, pravastatin, bezafibrate, ciprofibrate, gemfibrozil, aspirin, acemetacin, sodium ozagrel, beraprost sodium, and tirofiban, or pharmaceutically acceptable salts thereof;
still preferably, the substance used for preventing and/or treating atherosclerosis or an atherosclerosis-related disease is water-soluble statins, preferably rosuvastatin, pravastatin or atorvastatin.

6. The liposome nanocarrier delivery system according to any one of claims 1 and 3-5, **characterized in that** in the liposome nanocarrier delivery system, the final concentration of the targeting ligand is 100±24 µg/mL.

7. The liposome nanocarrier delivery system according to any one of claims 1-6, **characterized in that** the liposome nanocarrier delivery system comprises one or more of the following definitions:
i) the atherosclerosis-related disease is one or more selected from the following: atherosclerosis, coronary atherosclerotic heart disease, cerebral atherosclerosis, peripheral vascular atherosclerosis, aortic dissection, hemangioma, thromboembolism, heart failure, and cardiogenic shock;
ii) the liposome carrier is selected from small unilamellar liposomes, large unilamellar liposomes or multilamellar liposomes;
iii) the average molecular weight of hyaluronic acid being from 12,000 to 16,000.

8. A method for preparing the liposome nanocarrier delivery system according to any one of claims 1-7, including a step of hydrating the liposome carrier by adding a salt solution and performing separation to form a solution acidity gradient between inside and outside of the liposomes.

9. The method for preparing the liposome nanocarrier delivery system according to claim 8, **characterized in that** the salt is a salt of weak acid and strong alkali;
preferably, the anionic portion of the salt is one or more selected from the following:
acetate, edetate, bicarbonate, hypochlorite, citrate, benzoate and gluconate; and/or
the cationic portion of the salt is one or more selected from the following: calcium ions, copper ions, nickel ions, barium ions, magnesium ions and zinc ions;
yet preferably, the salt is one or more selected from the following: calcium acetate, calcium bicarbonate, magnesium citrate and copper gluconate.

10. The method for preparing the liposome nanocarrier delivery system according to claim 8 or 9, **characterized in that** the method comprises the following steps:
(1) dissolving the liposome carrier material in a solvent selected from the following: chloroform, ethanol, methanol, methylene chloride, acetone, toluene, diethyl ether, ethyl acetate, methyl acetate, acetonitrile, and dichloroethane;
(2) adding a salt solution that forms a solution acidity gradient between inside and outside of the liposomes to the product obtained in step (1) to hydrate and disperse it into a crude lipid solution;
(3) reducing the particle size of the crude lipid solution obtained in step (2) to obtain refined liposomes;
(4) purifying the refined liposome solution obtained in step (3) to remove external ions, and forming an acidity gradient between inside and outside of the liposomes; and
(5) adding a solution of the substance to be encapsulated to the liposome solution obtained in step (4), and performing incubation to allow the drug to enter the interior of the liposomes to obtain the liposome nanocarrier delivery system.

11. The method according to claim 10, **characterized in that** the method comprises one or more of the following definitions:
i) the step (1) further comprises a step of removing the solvent by evaporation to make the liposomes form a lipid membrane;
preferably, the evaporation temperature is 40-80°C, preferably 50-70°C, yet preferably 50-60°C;
yet preferably, the evaporation method is rotary evaporation; and
still preferably, the rotary evaporation is performed at a speed of 50-200 r/min, preferably 70-120 r/min, yet preferably 80-100 r/min;
ii) in step (1), the phospholipid is at a concentration of 0.1-500 mg/mL, preferably 0.1-200 mg/mL, yet preferably 0.2-100 mg/mL; and/or
the cholesterol is at a concentration of 0.05-200 mg/mL, preferably 0.1-100 mg/mL, yet preferably 0.1-50 mg/mL;
iii) in step (2), the salt solution has a concentration of 100-500 mM, preferably 200-300 mM, yet preferably 200-250 mM; and/or
the hydration temperature is 30-90°C, preferably40-80°C, yet preferably45-65°C;
iv) in step (3), the method for reducing the particle size is extruding the crude lipid solution through a filter membrane;
preferably, the filter membrane is a polycarbonate membrane;
yet preferably, the filter membrane has a pore size of 30-800 nm, such as 50-400 nm, such as 100-200 nm, such as 30 nm, 50 nm, 100 nm, 200 nm, 400 nm, 600 nm or 800 nm, preferably 100-150 nm;
v) in step (4), the method for removing external ions is one or more selected from the following: separation through an ultrafiltration membrane pack, dialysis separation and ion exchange;
vi) in step (5), the solution of the substance to be encapsulated is at a concentration of 0.01-100 mg/mL, preferably 0.05-50 mg/mL, yet preferably 0.1-20 mg/mL, still preferably 0.1-10 mg/mL.

12. The method according to any one of claims 10-11, **characterized in that** in step (5), the incubation temperature is 20-80°C, preferably 40-70°C, yet preferably 55-65°C.

13. The method according to any one of claims 10-12, **characterized in that** the method further comprises the following step:
(6) co-incubating the targeting ligand with the liposome nanocarrier delivery system obtained in step (5) to obtain a modified liposome nanocarrier delivery system.

14. A medicine, **characterized in that** the medicine comprises the nanocarrier delivery system according to any one of claims 1-7 or the nanocarrier delivery system prepared by the method according to any one of claims 8-13, and a pharmaceutically acceptable carrier.

15. The nanocarrier delivery system according to any one of claims 1-7, the nanocarrier delivery system prepared by the method according to any one of claims 8-13, and the medicine according to claim 13, for use in preventing and/or treating a disease, wherein the disease is atherosclerosis or an atherosclerosis-related disease;
wherein preferably, the disease is one or more selected from the following: atherosclerosis, coronary atherosclerotic heart disease (including acute coronary syndrome, asymptomatic myocardial ischemia-latent coronary heart disease, angina pectoris, myocardial infarction, ischemic heart disease, sudden death, and in-stent restenosis), cerebral atherosclerosis (including cerebral ischemic stroke and hemorrhagic apoplexy), peripheral vascular atherosclerosis (including carotid atherosclerosis, vertebral atherosclerosis, subclavian atherosclerosis, occlusive peripheral atherosclerosis, retinal atherosclerosis, renal atherosclerosis, lower extremity atherosclerosis, upper extremity atherosclerosis, mesenteric atherosclerosis and atherosclerotic impotence), aortic dissection, hemangioma, thromboembolism, heart failure, and cardiogenic shock.

16. The nanocarrier delivery system according to any one of claims 1-7, the nanocarrier delivery system prepared by the method according to any one of claims 8-13, and the medicine according to claim 14, for use in treating vascular plaques;
preferably, for use in treating arterial plaques;
yet preferably, for use in curbing the progression of arterial plaques, reversing arterial plaques and/or reducing the volume of arterial plaques.

## Patentansprüche

1. Aktiv verkapselndes Liposomen-Nanoträger-Abgabesystem, **dadurch gekennzeichnet, dass** das Liposomen-Nanoträger-Abgabesystem einen Liposomenträger und eine Substanz einschließt, die zum Verhindern und/oder Behandeln von Atherosklerose oder einer Atherosklerose-bedingten Erkrankung verwendet wird und von dem Liposomenträger aktiv verkapseln wird;
wobei:
- Materialien des Liposomenträgers Phospholipid und Cholesterin umfassen;
- das Liposomen-Nanoträger-Abgabesystem mit einem Zielliganden modifiziert, bei dem es sich um Hyaluronsäure und DSPE-PEG2000-NH₂ handelt, die über eine Amidbindung gekoppelt sind; und
- im Liposomen-Nanoträger-Abgabesystem die Endkonzentration des Zielliganden 10 bis 200 µg/ml beträgt.

2. Liposomen-Nanoträger-Abgabesystem nach Anspruch 1, **dadurch gekennzeichnet, dass** in dem Liposomen-Nanoträger-Abgabesystem die Endkonzentration des Zielliganden 10 bis 100 µg/ml beträgt.

3. Liposomen-Nanoträger-Abgabesystem nach Anspruch 1 oder 2, **dadurch gekennzeichnet, dass** das Phospholipid eines oder mehrere ausgewählt aus den folgenden ist: hydriertes Soja-Phosphatidylcholin, Distearoylphosphatidylcholin, Distearoylphosphatidylethanolamin, Polyethylenglykol-Distearoylphosphatidylethanolamin, Distearoylphosphatidylglycerin, Distearoylphosphatidylserin, Dioleoylphosphatidylcholin, Dioleoylphosphatidylethanolamin, Dioleoylphosphatidylserin, Dioleoylphosphatidylethanolamin-Polyethylenglykol, Dicinnamoylphosphatidylcholin, Dicinnamoylphosphatidylethanolamin, Dicinnamoylphosphatidylserin, Dicinnamoylphosphatidylethanolamin-Polyethylenglykol, Dipalmitoylphosphatidylcholin, Dipalmitoylphosphatidylethanolamin, Dipalmitoylphosphatidylserin, Dipalmitoylphosphatidylethanolamin-Polyethylenglykol, Dierucoylphosphatidylcholin, Dierucoylphosphatidylethanolamin, Dierucylphosphatidylserin, Dierucylphosphatidylethanolamin-Polyethylenglykol, Sphingomyelin, Eiphosphatidylcholin, Eiphosphatidylethanolamin, und Eiphosphatidylglycerol.

4. Liposomen-Nanoträger-Abgabesystem nach einem der Ansprüche 1 bis 3, **dadurch gekennzeichnet, dass** das Massenverhältnis der Substanz, die zum Verhindern und/oder Behandeln von Atherosklerose oder einer Atherosklerose-bedingten Erkrankung verwendet wird, zum Liposomenträger 1:0,1 bis 50, vorzugsweise 1:0,2 bis 30, noch bevorzugter 1:0,5 bis 20 beträgt.

5. Liposomen-Nanoträger-Abgabesystem nach einem der Ansprüche 1 bis 4, **dadurch gekennzeichnet, dass** die Substanz, die zum Verhindern und/oder Behandeln von Atherosklerose oder einer Atherosklerose-bedingten Erkrankung verwendet wird, eine schwach saure Substanz ist;
vorzugsweise handelt es sich bei der Substanz, die zum Verhindern und/oder Behandeln von Atherosklerose oder einer Atherosklerose-bedingten Erkrankung verwendet wird, um eine oder mehrere ausgewählt aus den folgenden: Statine, Fibrate, Thrombozytenaggregationshemmer, PCSK9-Hemmer, Antikoagulanzien, Angiotensin-Converting-Enzym-Hemmer, Calciumion-Antagonisten, MMP-Hemmer, β-Rezeptorblocker, Glucocorticoide und pharmazeutisch annehmbare Salze davon; vorzugsweise handelt es sich bei der Substanz, die zum Verhindern und/oder Behandeln von Atherosklerose oder einer Atherosklerose-bedingten Erkrankung verwendet wird, um eine oder mehrere ausgewählt aus den folgenden: Lovastatin, Atorvastatin, Rosuvastatin, Simvastatin, Fluvastatin, Pitavastatin, Pravastatin, Bezafibrat, Ciprofibrat, Gemfibrozil, Aspirin, Acemetacin, Natriumozagrel, Beraprost-Natrium und Tirofiban oder pharmazeutisch annehmbare Salze davon;
noch bevorzugter handelt es sich bei der Substanz, die zum Verhindern und/oder Behandeln von Atherosklerose oder einer Atherosklerose-bedingten Erkrankung verwendet wird, um wasserlösliche Statine, bevorzugt Rosuvastatin, Pravastatin oder Atorvastatin.

6. Liposomen-Nanoträger-Abgabesystem nach einem der Ansprüche 1 und 3 bis 5, **dadurch gekennzeichnet, dass** in dem Liposomen-Nanoträger-Abgabesystem die Endkonzentration des Zielliganden 100±24 µg/ml beträgt.

7. Liposomen-Nanoträger-Abgabesystem nach einem der Ansprüche 1 bis 6, **dadurch gekennzeichnet, dass** das Liposomen-Nanoträger-Abgabesystem eine oder mehrere der folgenden Definitionen umfasst:
i) die Atherosklerose-bedingte Erkrankung ist eine oder mehrere ausgewählt aus den folgenden: Atherosklerose, koronare atherosklerotische Herzerkrankung, zerebrale Atherosklerose, periphere vaskuläre Atherosklerose, Aortendissektion, Hämangiom, Thromboembolie, Herzinsuffizienz und kardiogener Schock;
ii) der Liposomenträger ist ausgewählt aus kleinen unilamellaren Liposomen, großen unilamellaren Liposomen oder multilamellaren Liposomen;
iii) das durchschnittliche Molekulargewicht der Hyaluronsäure beträgt von 12.000 bis 16.000.

8. Verfahren zum Herstellen des Liposomen-Nanoträger-Abgabesystems nach einem der Ansprüche 1 bis 7, einschließlich eines Schritts des Hydratisierens des Liposomenträgers durch Hinzufügen einer Salzlösung und Durchführung einer Trennung zum Bilden eines Lösungssäuregradienten zwischen innerhalb und außerhalb der Liposomen.

9. Verfahren zum Herstellen des Liposomen-Nanoträger-Abgabesystems nach Anspruch 8, **dadurch gekennzeichnet, dass** das Salz ein Salz einer schwachen Säure und eines starken Alkalis ist;
bevorzugt ist der anionische Teil des Salzes einer oder mehrere ausgewählt aus den folgenden:
Acetat, Edetat, Bicarbonat, Hypochlorit, Citrat, Benzoat und Gluconat; und/oder der kationische Teil des Salzes eines oder mehrere ist ausgewählt aus den folgenden: Calciumionen, Kupferionen, Nickelionen, Bariumionen, Magnesiumionen und Zinkionen; vorzugsweise ist das Salz eines oder mehrere ausgewählt aus den folgenden: Calciumacetat, Calciumbicarbonat, Magnesiumcitrat und Kupfergluconat.

10. Verfahren zum Herstellen des Liposomen-Nanoträger-Abgabesystems nach Anspruch 8 oder 9, **dadurch gekennzeichnet, dass** das Verfahren die folgenden Schritte umfasst:
(1) Auflösen des Liposomenträgermaterials in einem Lösungsmittel, das ausgewählt ist aus den folgenden: Chloroform, Ethanol, Methanol, Methylenchlorid, Aceton, Toluol, Diethylether, Ethylacetat, Methylacetat, Acetonitril und Dichlorethan;
(2) Hinzufügen einer Salzlösung, die einen Lösungssäuregradienten zwischen innerhalb und außerhalb der Liposomen bildet, zu dem in Schritt (1) erhaltenen Produkt, um es zu hydratisieren und in eine rohe Lipidlösung zu dispergieren;
(3) Reduzieren der Partikelgröße der in Schritt (2) erhaltenen rohen Lipidlösung, um verfeinerte Liposomen zu erhalten;
(4) Reinigen der in Schritt (3) erhaltenen verfeinerten Liposomenlösung, um externe Ionen zu entfernen und einen Säuregradienten zwischen innerhalb und außerhalb der Liposomen zu bilden; und
(5) Hinzufügen einer Lösung der zu verkapselnden Substanz zu der in Schritt (4) erhaltenen Liposomenlösung und Durchführen einer Inkubation, um dem Arzneimittel zu ermöglichen, in das Innere der Liposomen einzutreten, um das Liposomen-Nanoträger-Abgabesystem zu erhalten.

11. Verfahren nach Anspruch 10, **dadurch gekennzeichnet, dass** das Verfahren eine oder mehrere der folgenden Definitionen umfasst:
i) der Schritt (1) umfasst ferner einen Schritt des Entfernens des Lösungsmittels durch Verdampfen, um die Liposomen eine Lipidmembran bilden zu lassen;
bevorzugt beträgt die Verdampfungstemperatur 40 bis 80 °C, bevorzugt 50 bis 70 °C, vorzugsweise 50 bis 60 °C;
vorzugsweise ist das Verdampfungsverfahren eine Rotationsverdampfung; und
noch bevorzugter wird die Rotationsverdampfung bei einer Drehzahl von 50 bis 200 U/min, bevorzugt 70 bis 120 U/min, vorzugsweise 80 bis 100 U/min, durchgeführt;
ii) in Schritt (1) liegt das Phospholipid in einer Konzentration von 0,1 bis 500 mg/ml, bevorzugt 0,1 bis 200 mg/ml, vorzugsweise 0,2 bis 100 mg/ml vor; und/oder das Cholesterin liegt in einer Konzentration von 0,05 bis 200 mg/ml, bevorzugt 0,1 bis 100 mg/ml, vorzugsweise 0,1 bis 50 mg/ml vor;
iii) in Schritt (2) weist die Salzlösung eine Konzentration von 100 bis 500 mM, bevorzugt 200 bis 300 mM, vorzugsweise 200 bis 250 mM auf; und/oder
die Hydratisierungstemperatur beträgt 30 bis 90 °C, bevorzugt 40 bis 80 °C, vorzugsweise 45 bis 65 °C;
iv) in Schritt (3) besteht das Verfahren zum Reduzieren der Partikelgröße im Extrudieren der rohen Lipidlösung durch eine Filtermembran;
bevorzugt ist die Filtermembran eine Polycarbonatmembran;
vorzugsweise weist die Filtermembran eine Porengröße von 30 bis 800 nm auf, wie zum Beispiel 50 bis 400 nm, wie zum Beispiel 100 bis 200 nm, wie zum Beispiel 30 nm, 50 nm, 100 nm, 200 nm, 400 nm, 600 nm oder 800 nm, bevorzugt 100 bis 150 nm;
v) in Schritt (4) ist das Verfahren zum Entfernen externer Ionen eines oder mehrere ausgewählt aus den folgenden: Trennung durch ein Ultrafiltrationsmembranpaket, Dialysetrennung und Ionenaustausch;
vi) in Schritt (5) weist die Lösung der zu verkapselnden Substanz eine Konzentration von 0,01 bis 100 mg/ml auf, bevorzugt 0,05 bis 50 mg/ml, vorzugsweise 0,1 bis 20 mg/ml, noch bevorzugter 0,1 bis 10 mg/ml.

12. Verfahren nach einem der Ansprüche 10 bis 11, **dadurch gekennzeichnet, dass** in Schritt (5) die Inkubationstemperatur 20 bis 80 °C, bevorzugt 40 bis 70 °C, vorzugsweise 55 bis 65 °C beträgt.

13. Verfahren nach einem der Ansprüche 10 bis 12, **dadurch gekennzeichnet, dass** das Verfahren ferner den folgenden Schritt umfasst:
(6) Coinkubieren des Zielliganden mit dem in Schritt (5) erhaltenen Liposomen-Nanoträger-Abgabesystem, um ein modifiziertes Liposomen-Nanoträger-Abgabesystem zu erhalten.

14. Medikament, **dadurch gekennzeichnet, dass** das Medikament das Nanoträger-Abgabesystem nach einem der Ansprüche 1 bis 7 oder das durch das Verfahren nach einem der Ansprüche 8 bis 13 hergestellte Nanoträger-Abgabesystem und einen pharmazeutisch annehmbaren Träger umfasst.

15. Nanoträger-Abgabesystem nach einem der Ansprüche 1 bis 7, durch das Verfahren nach einem der Ansprüche 8 bis 13 hergestelltes Nanoträger-Abgabesystem und Medikament nach Anspruch 13 zur Verwendung zum Verhindern und/oder Behandeln einer Erkrankung, wobei es sich bei der Erkrankung um Atherosklerose oder eine Atherosklerose-bedingte Erkrankung handelt;
wobei es sich bei der Erkrankung bevorzugt um eine oder mehrere ausgewählt aus den folgenden handelt: Atherosklerose, koronare atherosklerotische Herzerkrankung (einschließlich akutes Koronarsyndrom, asymptomatische Myokardischämie - latente koronare Herzerkrankung, Angina pectoris, Myokardinfarkt, ischämische Herzerkrankung, plötzlicher Tod und In-Stent-Restenose), zerebrale Atherosklerose (einschließlich zerebraler ischämischer Schlaganfall und hämorrhagischer Schlaganfall), periphere vaskuläre Atherosklerose (einschließlich Karotis-Atherosklerose, Vertebral-Atherosklerose, Subclavia-Atherosklerose, okklusive periphere Atherosklerose, retinale Atherosklerose, renale Atherosklerose, Atherosklerose der unteren Extremitäten, Atherosklerose der oberen Extremitäten, mesenteriale Atherosklerose und atherosklerotische Impotenz), Aortendissektion, Hämangiom, Thromboembolie, Herzinsuffizienz und kardiogener Schock.

16. Nanoträger-Abgabesystem nach einem der Ansprüche 1 bis 7, durch das Verfahren nach einem der Ansprüche 8 bis 13 hergestelltes Nanoträger-Abgabesystem und Medikament nach Anspruch 14 zur Verwendung beim Behandeln von Gefäßplaques; bevorzugt zur Verwendung beim Behandeln von arteriellen Plaques;
vorzugsweise zur Verwendung beim Eindämmen des Fortschreitens von arteriellen Plaques, Rückbilden von arteriellen Plaques und/oder Reduzieren des Volumens von arteriellen Plaques.

## Revendications

1. Système d'administration par nanovecteurs liposomiques à encapsulation active, **caractérisé en ce que** le système d'administration par nanovecteurs liposomiques inclut un vecteur liposomique et une substance utilisée pour prévenir et/ou traiter l'athérosclérose ou une maladie liée à l'athérosclérose encapsulée activement par le vecteur liposomique ;
dans lequel :
- des matériaux du vecteur liposomique comprennent un phospholipide et du cholestérol ;
- le système d'administration par nanovecteurs liposomiques est modifié avec un ligand de ciblage qui est de l'acide hyaluronique et du DSPE-PEG2000-NH₂ couplé par une liaison amide ; et
- dans le système d'administration par nanovecteurs liposomiques, la concentration finale du ligand de ciblage est 10 à 200 µg/mL.

2. Système d'administration par nanovecteurs liposomiques selon la revendication 1, **caractérisé en ce que**, dans le système d'administration par nanovecteurs liposomiques, la concentration finale du ligand de ciblage est 10 à 100 µg/mL.

3. Système d'administration par nanovecteurs liposomiques selon la revendication 1 ou 2, **caractérisé en ce que** le phospholipide est un ou plusieurs sélectionnés parmi les suivants : phosphatidylcholine de soja hydrogénée, distéaroylphosphatidylcholine, distéaroylphosphatidyléthanolamine, polyéthylène glycol-distéaroylphosphatidyléthanolamine, distéaroylphosphatidylglycérol, distéaroylphosphatidylsérine, dioléoylphosphatidylcholine, dioléoylphosphatidyléthanolamine, dioléoylphosphatidylsérine, dioléoylphosphatidyléthanolamine-polyéthylène glycol, dicinnamoylphosphatidylcholine, dicinnamoylphosphatidyléthanolamine, dicinnamoylphosphatidylsérine, dicinnamoylphosphatidyléthanolamine-polyéthylène glycol, dipalmitoylphosphatidylcholine, dipalmitoylphosphatidyléthanolamine, dipalmitoylphosphatidylsérine, dipalmitoylphosphatidyléthanolamine-polyéthylène glycol, diérucoylphosphatidylcholine, diérucoylphosphatidyléthanolamine, diérucylphosphatidylsérine, diérucylphosphatidyléthanolamine-polyéthylène glycol,
sphingomyéline, phosphatidylcholine d'œuf, phosphatidyléthanolamine d'œuf et phosphatidylglycérol d'œuf.

4. Système d'administration par nanovecteurs liposomiques selon l'une quelconque des revendications 1 à 3, **caractérisé en ce que** le rapport en masse de la substance utilisée pour prévenir et/ou traiter l'athérosclérose ou une maladie liée à l'athérosclérose sur le vecteur liposomique est 1:0,1 à 50, de préférence 1:0,2 à 30, de préférence encore 1:0,5 à 20.

5. Système d'administration par nanovecteurs liposomiques selon l'une quelconque des revendications 1 à 4, **caractérisé en ce que** la substance utilisée pour prévenir et/ou traiter l'athérosclérose ou une maladie liée à l'athérosclérose est une substance faiblement acide ;
de préférence encore, la substance utilisée pour prévenir et/ou traiter l'athérosclérose ou une maladie liée à l'athérosclérose est une ou plusieurs sélectionnées parmi les suivantes : statines, fibrates, médicaments antiplaquettaires, inhibiteurs de PCSK9, médicaments anticoagulants, inhibiteurs d'enzyme de conversion d'angiotensine, antagonistes d'ions calcium, inhibiteurs de MMP, bloqueurs de récepteur β, glucocorticoïde, et sels pharmaceutiquement acceptables de ceux-ci ;
de préférence encore, la substance utilisée pour prévenir et/ou traiter l'athérosclérose ou une maladie liée à l'athérosclérose est une ou plusieurs sélectionnées parmi les suivantes : la lovastatine, l'atorvastatine, la rosuvastatine, la simvastatine, la fluvastatine, la pitavastatine, la pravastatine, le bézafibrate, le ciprofibrate, le gemfibrozil, l'aspirine, l'acémétacine, l'ozagrel sodique, le béraprost sodique et le tirofiban, ou des sels pharmaceutiquement acceptables de ceux-ci ;
de manière encore préférée, la substance utilisée pour prévenir et/ou traiter l'athérosclérose ou une maladie liée à l'athérosclérose est des statines hydrosolubles, de préférence de la rosuvastatine, de la pravastatine ou de l'atorvastatine.

6. Système d'administration par nanovecteurs liposomiques selon l'une quelconque des revendications 1 et 3 à 5, **caractérisé en ce que** dans le système d'administration par nanovecteurs liposomiques, la concentration finale du ligand de ciblage est de 100±24 µg/mL.

7. Système d'administration par nanovecteurs liposomiques selon l'une quelconque des revendications 1 à 6, **caractérisé en ce que** le système d'administration par nanovecteurs liposomiques comprend une ou plusieurs des définitions suivantes :
i) la maladie liée à l'athérosclérose est une ou plusieurs parmi les suivantes : athérosclérose, cardiopathie athérosclérotique coronarienne, athérosclérose cérébrale, athérosclérose vasculaire périphérique, dissection aortique, hémangiome, thromboembolie, insuffisance cardiaque et choc cardiogénique ;
ii) le vecteur liposomique est sélectionné parmi les petits liposomes unilamellaires, les grands liposomes unilamellaires ou les liposomes multilamellaires ;
iii) le poids moléculaire moyen de l'acide hyaluronique étant de 12 000 à 16 000.

8. Procédé de préparation du système d'administration par nanovecteurs liposomiques selon l'une quelconque des revendications 1 à 7, incluant une étape d'hydratation du vecteur liposomique en ajoutant une solution saline et en réalisant une séparation pour former un gradient d'acidité de solution entre l'intérieur et l'extérieur des liposomes.

9. Procédé de préparation du système d'administration par nanovecteurs liposomiques selon la revendication 8, **caractérisé en ce que** le sel est un sel d'acide faible et d'alcali fort ;
de préférence, la portion anionique du sel est un ou plusieurs sélectionnés parmi les suivants :
acétate, édétate, bicarbonate, hypochlorite, citrate, benzoate et gluconate ; et/ou la portion cationique du sel est un ou plusieurs sélectionnés parmi les suivants : ions calcium, ions cuivre, ions nickel, ions baryum, ions magnésium et ions zinc ;
de préférence encore, le sel est un ou plusieurs sélectionnés parmi les suivants : acétate de calcium, bicarbonate de calcium, citrate de magnésium et gluconate de cuivre.

10. Procédé de préparation du système d'administration par nanovecteurs liposomiques selon la revendication 8 ou 9, **caractérisé en ce que** le procédé comprend les étapes suivantes :
(1) la dissolution du matériau de vecteur liposomique dans un solvant sélectionné parmi les suivants : chloroforme, éthanol, méthanol, chlorure de méthylène, acétone, toluène, éther diéthylique, acétate d'éthyle, acétate de méthyle, acétonitrile et dichloroéthane ;
(2) l'ajout d'une solution saline qui forme un gradient d'acidité de solution entre l'intérieur et l'extérieur des liposomes au produit obtenu à l'étape (1) pour l'hydrater et le disperser dans une solution lipidique brute ;
(3) la réduction de la taille de particules de la solution lipidique brute obtenue à l'étape (2) pour obtenir des liposomes raffinés ;
(4) la purification de la solution de liposome raffinée obtenue à l'étape (3) pour éliminer les ions externes, et la formation d'un gradient d'acidité entre l'intérieur et l'extérieur des liposomes ; et
(5) l'ajout d'une solution de la substance à encapsuler à la solution de liposomes obtenue à l'étape (4), et la réalisation d'une incubation pour permettre au médicament de pénétrer à l'intérieur des liposomes pour obtenir le système d'administration par nanovecteurs liposomiques.

11. Procédé selon la revendication 10, **caractérisé en ce que** le procédé comprend une ou plusieurs des définitions suivantes :
i) l'étape (1) comprend en outre une étape d'élimination du solvant par évaporation pour que les liposomes forment une membrane lipidique ;
de préférence, la température d'évaporation est 40 à 80 °C, de préférence 50 à 70 °C, de préférence encore 50 à 60 °C ;
de préférence encore, le procédé d'évaporation est une évaporation rotative ; et
de manière encore préférée, l'évaporation rotative est réalisée à une vitesse de 50 à 200 trs/min, de préférence 70 à 120 trs/min, de préférence encore 80 à 100 trs/min ;
ii) à l'étape (1), le phospholipide est à une concentration de 0,1 à 500 mg/mL, de préférence 0,1 à 200 mg/mL, de préférence encore 0,2 à 100 mg/mL ; et/ou le cholestérol est à une concentration de 0,05 à 200 mg/mL, de préférence 0,1 à 100 mg/mL, de préférence encore 0,1 à 50 mg/mL ;
iii) à l'étape (2), la solution saline a une concentration de 100 à 500 mM, de préférence 200 à 300 mM, de préférence encore 200 à 250 mM ; et/ou
la température d'hydratation est de 30 à 90 °C, de préférence 40 à 80 °C, de préférence encore 45 à 65 °C ;
iv) à l'étape (3), le procédé de réduction de la taille des particules est l'extrusion de la solution lipidique brute à travers une membrane filtrante ;
de préférence, la membrane filtrante est une membrane en polycarbonate ; de préférence encore, la membrane filtrante a une taille de pores de 30 à 800 nm, telle que 50 à 400 nm, telle que 100 à 200 nm, telle que 30 nm, 50 nm, 100 nm, 200 nm, 400 nm, 600 nm ou 800 nm, de préférence 100 à 150 nm ;
v) à l'étape (4), le procédé d'élimination d'ions externes est un ou plusieurs sélectionnés parmi les suivants : séparation à travers un bloc de membranes d'ultrafiltration, séparation par dialyse et échange d'ions ;
vi) à l'étape (5), la solution de la substance à encapsuler est à une concentration de 0,01 à 100 mg/mL, de préférence 0,05 à 50 mg/mL, de préférence encore 0,1 à 20 mg/mL, de manière encore préférée 0,1 à 10 mg/mL

12. Procédé selon l'une quelconque des revendications 10 à 11, **caractérisé en ce qu'**à l'étape (5), la température d'incubation est de 20 à 80 °C, de préférence 40 à 70 °C, de préférence encore 55 à 65 °C.

13. Procédé selon l'une quelconque des revendications 10 à 12, **caractérisé en ce que** le procédé comprend en outre l'étape suivante :
(6) la co-incubation du ligand de ciblage avec le système d'administration par nanovecteurs liposomiques obtenu à l'étape (5) pour obtenir un système d'administration par nanovecteurs liposomiques modifié.

14. Médicament, **caractérisé en ce que** le médicament comprend le système d'administration par nanovecteurs selon l'une quelconque des revendications 1 à 7 ou le système d'administration par nanovecteurs préparé par le procédé selon l'une quelconque des revendications 8 à 13, et un vecteur pharmaceutiquement acceptable.

15. Système d'administration par nanovecteurs selon l'une quelconque des revendications 1 à 7, le système d'administration par nanovecteurs étant préparé grâce au procédé selon l'une quelconque des revendications 8 à 13, et le médicament selon la revendication 13, pour une utilisation dans la prévention et/ou le traitement d'une maladie, dans lequel la maladie est l'athérosclérose ou une maladie liée à l'athérosclérose ;
dans lequel de préférence, la maladie est une ou plusieurs sélectionnées parmi les suivantes : l'athérosclérose, la cardiopathie athérosclérotique coronarienne (y compris le syndrome coronarien aigu, l'ischémie myocardique asymptomatique-coronarienne latente, l'angine de poitrine, l'infarctus du myocarde, la cardiopathie ischémique, la mort subite et la resténose intra-stent), l'athérosclérose cérébrale (y compris l'accident ischémique cérébral et l'apoplexie hémorragique), l'athérosclérose vasculaire périphérique (y compris l'athérosclérose carotidienne, l'athérosclérose vertébrale, l'athérosclérose sous-clavière, l'athérosclérose périphérique occlusive, l'athérosclérose rétinienne, l'athérosclérose rénale, l'athérosclérose des membres inférieurs, l'athérosclérose des membres supérieurs, l'athérosclérose mésentérique et l'impuissance athérosclérotique), la dissection aortique, l'hémangiome, la thromboembolie, l'insuffisance cardiaque et le choc cardiogénique.

16. Système d'administration par nanovecteurs selon l'une quelconque des revendications 1 à 7, le système d'administration par nanovecteurs étant préparé par le procédé selon l'une quelconque des revendications 8 à 13, et le médicament selon la revendication 14, pour une utilisation dans le traitement de plaques vasculaires ;
de préférence pour une utilisation dans le traitement de plaques artérielles ;
de préférence encore, pour une utilisation visant à freiner la progression de plaques artérielles, à faire régresser les plaques artérielles et/ou à réduire le volume de plaques artérielles.
